# EUROPÄISCHE PATENTSCHRIFT

(11) **EP 2 529 005 B1**
(45) Veröffentlichungstag und Bekanntmachung des Hinweises auf die Patenterteilung: **21.09.2016**
(21) Anmeldenummer: 11701104.9
(22) Anmeldetag: 25.01.2011
(51) Int. Cl.: C12N 5/00, C08B 37/00, C08J 3/075, C08L 5/04, A61K 31/734, A61P 25/28

(54) **HYDROGELEN AUF BASIS GULURONSÄURE- UND/ODER MANNURONSÄUREHALTIGER POLYSACCHARIDE ZUR BEHANDLUNG VON BESCHÄDIGUNGEN DES NERVENSYSTEMS, ZUR FÖRDERUNG DES NERVENWACHSTUMS, ZUR BEHANDLUNG VON NEURODEGENERATIVEN ERKRANKUNGEN UND ZUR KULTIVIERUNG VON NEURONEN**
HYDROGELS ON THE BASIS OF GULURONIC ACID- AND/OR MANNURONIC ACID-CONTAINING POLYSACCHARIDES FOR TREATING DAMAGE TO THE NERVOUS SYSTEM, PROMOTING NERVE GROWTH, TREATING NEURODEGENERATIVE DISEASES AND CULTIVATING NEURONS
HYDROGELS À BASE DE POLYSACCHARIDES CONTENANT DE L'ACIDE GULURONIQUE ET/OU DE L'ACIDE MANNURONIQUE POUR LE TRAITEMENT DES LÉSIONS DU SYSTÈME NERVEUX, POUR LA STIMULATION DE LA CROISSANCE NERVEUSE, POUR LE TRAITEMENT DES MALADIES NEURODÉGÉNÉRATIVES ET POUR LA MISE EN CULTURE DES NEURONES

(30) Priorität: 25.01.2010 DE 102010001179
(43) Veröffentlichungstag der Anmeldung: 05.12.2012
(73) Patentinhaber: Technische Universität Dresden, 01062 Dresden (DE)
(72) Erfinder: MATYASH, Marina, 13187 Berlin (DE); IKONOMIDOU, Hrissanthi, 13505 Berlin (DE); DESPANG, Florian, 01099 Dresden (DE); GELINSKY, Michael, 01662 Meißen (DE)
(74) Vertreter: Kailuweit & Uhlemann Patentanwälte Partnerschaft mbB
(86) Internationale Anmeldenummer: PCT/EP2011/050962
(87) Internationale Veröffentlichungsnummer: WO 2011/089261

(56) Entgegenhaltungen:
- EP-A1- 0 048 123
- WO-A1-2010/029433
- WO-A1-2010/088775
- WO-A2-2007/144389
- US-A- 3 386 921
- US-A- 5 563 186
- US-A- 5 944 754
- US-A1- 2006 247 165
- US-A1- 2009 075 933
- ENAMI J ET AL: "GROWTH OF NERVE CELLS CULTURED IN ALGINATE-COATED COLLAGEN GEL FIBERS", JAPANESE JOURNAL OF PHYSIOLOGY, Bd. 40, Nr. SUPPL, 1990, Seite S20, XP8135038, & 67TH ANNUAL MEETING OF THE PHYSIOLOGICAL SOCIETY OF JAPAN, MIYAZAKI, JAPAN, APRIL 3-5, 1990. JPN J P ISSN: 0021-521X

## Beschreibung

Die Erfindung betrifft die Verwendung eines Hydrogels, welches *α*-L-Guluronsäure (GulUA) und/oder *β*-D-Mannuronsäure (ManUA) haltige Oligo- oder Polysaccharide enthält, zur Behandlung von Beschädigungen des Nervensystems, zur Förderung des Nervenwachstums bzw. Neuritenwachstums, zur Prophylaxe von Nervenschädigungen bei chirurgischen Eingriffen und zur Behandlung und Prophylaxe von neurodegenerativen Erkrankungen. Die Erfindung eignet sich insbesondere zur Behandlung von Beschädigungen des Nervensystems, wie z. B. bei einer Querschnittslähmung oder der Unterbrechung peripherer Nerven. Desweiteren betrifft die Erfindung die Kultivierung von Neuronen auf Oberflächen (z. B. in Zellkulturgefäßen aller Art), welche mit obigem Hydrogel beschichtet worden sind.

Die beiden Uronsäuren α-L-Guluronsäure (GulUA) und β-D-Mannuronsäure (ManUA) sind Hauptbestandteile von Alginat, einem natürlichen linearen Polysaccharid, in welchem GulUA und ManUA 1,4-glycosidisch in wechselndem Verhältnis zu linearen Ketten verbunden sind. Alginat wird von Braunalgen und von einigen Bakterien (z. B. Azotobacter) gebildet. In der Alge stellt es das strukturgebende Element der Zellwände dar. Die interzelluläre Gelmatrix verleiht der Alge sowohl Flexibilität, als auch Festigkeit. Alginat ist u.a. ein Nebenprodukt bei der Gewinnung von Jod aus Meeresalgen im Nassverfahren. Es wird allerdings auch direkt für die Verwendung in der Lebensmittel-, sowie der Pharma-, und Kosmetikindustrie aus den Braunalgen extrahiert. Alginat findet vor allem als Verdickungs- oder Geliermittel Verwendung.

Es ist bekannt, dass wässrige Alginatsole (also kolloidale Lösungen von Alginaten) in Gegenwart von zwei- oder mehrwertigen Metallionen (wie z. B. Calciumionen) Hydrogele bilden. Aus dem Stand der Technik ist die Verwendung von Hydrogelen aus Alginaten prinzipiell zu zwei unterschiedlichen Zwecken bekannt. Zum einen werden Alginate als Schutzstrukturen eingesetzt, die eine räumliche Abgrenzung ermöglichen. In der biomedizinischen Forschung werden derartige Alginat-basierte Hydrogele vor allem zur Einkapselung von Zellen verwendet (gezeigt z. B. für Inselzellen der Bauchspeicheldrüse und für Knorpelzellen). Die aus dem Stand der Technik bekannten Alginat-basierten Hydrogele werden durch Zugabe hoher Calciumionenkonzentrationen gebildet (100 mmol/l und mehr).

Zum anderen werden Alginate auch als dreidimensionale Trägerstrukturen (Scaffolds) für die Kultivierung von Zellen sowie als Gewebeersatzmaterial im Sinne des Tissue Engineering eingesetzt. Hierbei werden aus Alginatsolen oder auch aus Alginat-Hydrogelen durch Verfahren wie jene der Gefriertrockung poröse, dreidimensionale Körper hergestellt, welche oftmals von chemischen Modifizierungen mit dem Zweck der Stabilisierung begleitet sind. Diese Alginat-Scaffolds werden als Trägerstruktur für lebende Zellen verwendet und besonders im Hinblick auf den Gelenkknorpel-Ersatz untersucht. Um das Zellwachstum anzuregen, werden die aus dem Stand der Technik bekannten Alginat-basierten Scaffolds mit Komponenten der extrazellulären Matrix (z. B. Fibronectin, Kollagen, Laminin), Wachstumsfaktoren, Adhäsions-Peptidsequenzen (z. B. RGD-Peptiden) oder auch Polysacchariden (Heparin) modifiziert. Eine besondere Variante kann durch das Verfahren der gerichteten, ionotropen Gelbildung von Alginatsolen erhalten werden. Durch das gerichtete Eindiffundieren von zwei- oder mehrwertigen Kationen bilden sich kanalförmige und parallel angeordnete Kapillarporen, welche das Alginat-Hydrogel in seiner ganzen Länge durchziehen können. Diese Kapillaren eignen sich nach dem Stand der Technik dazu, ein gerichtetes Zellwachstum oder das gerichtete Auswachsen von Zellfortsätzen zu begünstigen.

Der Einsatz von Alginaten zur Einkapselung von lebenden Zellen ist beispielsweise von Purcell et al. (Tissue Engin. Part C, (15) 2009, S. 1-10) beschrieben. In dieser Schrift wurden neurale Stammzellen (NSC) zur Stabilisierung in Alginatgelen verkapselt. Dies erfolgt durch Eintropfen oder Einsprühen von in einem Alginatsol suspendierten NSC in eine 100 mmol/1 (mM) CaCl₂-Lösung. Die so gebildeten Hydrogele formen kleine Kugeln, in welche die NSC verkapselt sind. In der Schrift wurde der Einfluss der Alginatzusammensetzung auf das Überleben, die Proliferation und die Freisetzung von Neurotrophinen durch NSC getestet. Guluronsäurereiche Alginate wurden in diesem Zusammenhang als besonders geeignet für die Freisetzung von Neurotrophinen beschrieben, wohingegen die Alginatzusammensetzung keinen Einfluss auf das Zellwachstum und die Proliferation von NSC hatte.

In der US2009/0214660 A1 werden Alginatmikrokapseln zur Stabilisierung durch Verkapselung von lebenden Zellen offenbart. Diese sind aus Alginaten mit einem hohen Gehalt an Mannuronsäure und einem Polykation mit einem Polydispersitätsindex kleiner als 1,5 (beispielsweise Poly-L-ornithin) zusammengesetzt, wobei hier die Kapsel aus einer Schicht Alginat, einer darüberliegenden Schicht des Polykations und einer abschließenden Alginatschicht besteht. Die Verlinkung des Alginats erfolgt mit einer CaCl₂-Konzentration von 15 bis 120 mmol/l.

US 5,876,742 offenbart zur Einkapselung von Zellen eine Zusammensetzung aus mehreren Schichten von Alginathydrogelen, welche jeweils unterschiedliche Gehalte multivalenter Kationen enthalten. Um die Zelle herum wird eine mehrschichtige Hülle aus Alginathydrogelen aufgebaut, wobei die äußere Hülle zur Stabilisierung hohe Konzentrationen multivalenter Kationen enthält. Die auf diese Weise verkapselten Zellen können beispielsweise transplantiert werden.

Suzuki et al. (Neurosci. Lett., (318) 2002, S. 121-124) beschreiben einen Alginatschwamm, basierend auf einem gefriergetrockneten und kovalent quervernetzten Alginatsol, welcher zur Regeneration von Nervenschädigungen v. a. bei Rückenmarksschäden im Tierversuch getestet wurde. Der Alginatschwamm wurde aus einem 1 %igen Natriumalginatsol (aus mannuronsäurereichem Alginat) durch kovalentes Quervernetzen mit Ethylendiamin und wasserlöslichem Carbodiimid hergestellt. Das so erhältliche kovalent quervernetzte Gel wurde abschließend mit Natriumchlorid und Calciumchlorid gewaschen und gefriergetrocknet, womit eine poröse, dreidimensionale (schwammartige) Struktur erzeugt wurde. Der implantierte Alginatschwamm konnte zu einem gerichteten Wachstum von Axonen von Nervenzellen beitragen.

Beim Einsatz von Alginaten zur Unterstützung des gerichteten Zellwachstums wird nach dem Stand der Technik das besondere Phänomen ausgenutzt, dass Alginat anisotrope Kapillarhydrogele (Anisotropie capillary hydrogels, ACH) ausbildet, wenn ein wässriges Sol eines Alginats und eine Lösung, die zwei- oder mehrwertige Metallionen enthält, übereinandergeschichtet werden und eine Verwirbelung vermieden wird (Thiele, H., Histolyse und Histogenese, Gewebe und ionotrope Gele, Prinzip einer Stukturbildung. 1967, Frankfurt: Akademische Verlagsgesellschaft). Verwendet werden die ACH beispielsweise von Prang et al. (Biomaterials, (27) 2006, S. 3560-3569) um durch die Kapillaren ein gerichtetes Wachstum der Axone von Nervenzellen und so den Heilungsprozess bei Rückenmarksschädigungen zu unterstützen. Die dafür verwendeten anisotropen Kapillarhydrogele, welche auf Alginat mit einem Anteil von etwa 70 % an Guluronsäure basieren, wurden hierzu mit einer 1 mol/l Cu(NO₃)₂-Lösung quervernetzt. Ein Alginat-Kapillargel, welches durch Eindiffundieren von Cu²⁺-Ionen hergestellt wurde ist auch in WO 2005/087287 offenbart.

US 2009/0010983 A1 offenbart Implantatmaterialien mit einem Polymerschaum als Trägerstruktur. Die Poren des Polymerschaums sind mit einem Polysaccharidgel, beispielsweise einem Alginatgel, gefüllt. Die Konzentration an multivalenten Kationen, die zur Bereitstellung des Hydrogels eingesetzt wird, beträgt mindestens etwa 45 mmol/l. Der Polymerschaum dient dabei als Stützstruktur zur Förderung des dreidimensionalen Zellwachstums verschiedener Zelltypen, welche im Hydrogel suspendiert werden, ehe sie in die Polymerschaummatrix eingebracht werden. Es wurde beobachtet, dass durch die Anwendung der derartig gestalteten dreidimensionalen Stützstruktur ein dreidimensionales Zellwachstum gefördert werden konnte.

Banerjee et al. (Biomaterials, (30) 2009, S. 4695-4699) offenbart ein Verfahren zur dreidimensionalen Kulivierung neuronaler Stammzellen in Alginathydrogelen. Dafür werden die neuronalen Stammzellen zur Kultivierung in das Alginathydrogel, welches Zusätze von Wachstumsfaktoren (FGF-2) enthält, eingebettet und die Differenzierung der Stammzellen in Neuronen analysiert. Die beste Proliferation der Stammzellen wurde in Alginathydrogelen mit 0,25 % Alginat, 10 mM CaCl₂ erzielt.

Andere Schriften offenbaren den Einsatz von Alginathydrogelen als Stützstrukturen zur Unterstützung des Knochenwachstums. Dafür werden Kapillar-Hydrogele aus Alginaten in Kombination mit Calciumphosphaten, wie beispielsweise Hydroxylapatit, eingesetzt (Dittrich R. et al., Adv. Sci. Techn., (49) 2006, S. 159-164; Bernhardt A. et al., J. Tissue Eng. Regen. Med., (3) 2009, S. 54-62).

Die WO 91/11205 offenbart eine Zusammensetzung zur Wundheilung enthaltend Alginate mit einem Mindestanteil von Mannuronsäure von 70 %. Diese dient zur Erleichterung der Wundheilung und unterstützt das Fibroblastenwachstum durch die Förderung der Freisetzung von Zytokinen wie Interleukinen 1 und 6, sowie Tumornekrosefaktor.

Bisher sind noch keine Lösungen bekannt, die dazu dienen, das Wachstum von Neuriten von Nervenzellen zu fördern bzw. die Kultivierung von Neuronen in Zellkulturgefäßen zu verbessern.

Aufgabe der Erfindung ist es daher, das Wachstum von Neuriten von Nervenzellen gezielt zu fördern und/oder die Adhäsion von Neuronen auf Oberflächen, insbesondere von Zellkulturgefäßen, zu steigern.

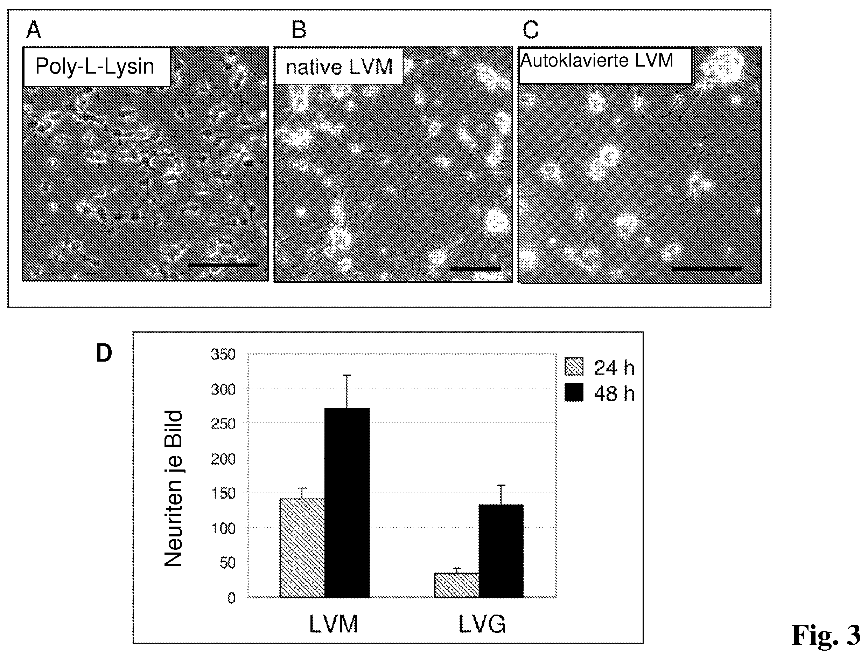

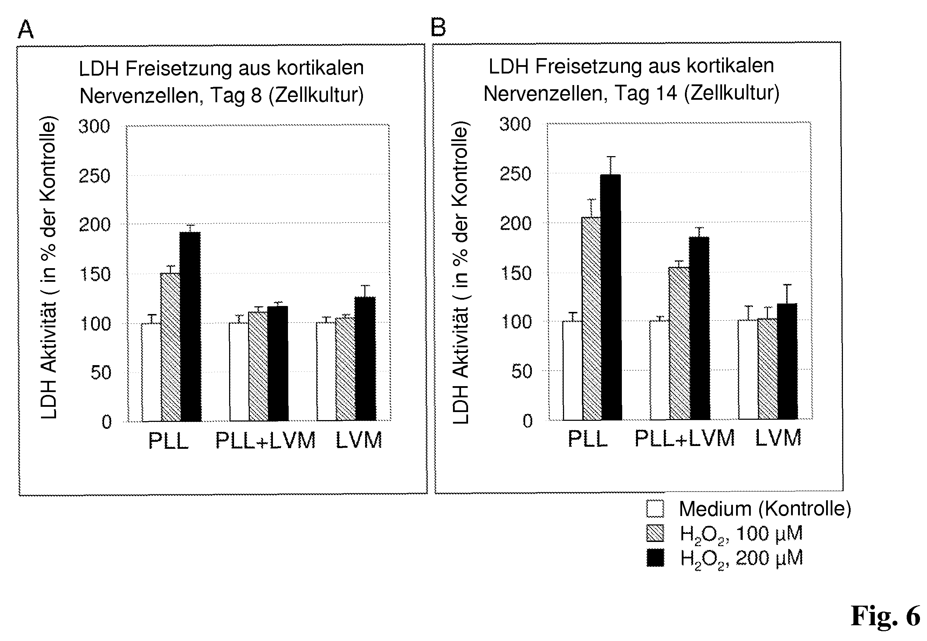

Die Erfinder haben überraschend gefunden, dass Polysaccharide, welche GulUA und/oder ManUA enthalten, die Adhäsion von Neuronen und das Wachstum von Neuriten fördern, sowie Nervenzellen vor oxidativem Stress schützen. Dies konnte besonders bei physiologischen Konzentrationen von multivalenten Kationen beobachtet werden (siehe **Fig. 3****,** **4****,** **5** **und** **6****).** Bei höheren Konzentrationen (z. B. 100 mmol/l CaCl₂- siehe **Fig. 6**) wird der Effekt jedoch nicht mehr beobachtet. Als Neuriten werden Fortsätze der Nervenzellen bezeichnet, welche dem Empfang oder der Weiterleitung der von der Nervenzelle ausgelösten Impulse dienen.

Diese Erkenntnis der Erfinder ermöglicht vorteilhaft die Anwendung dieser Polysaccharide bzw. Hydrogele, welche diese enthalten, im Bereich der Neurowissenschaften und der Neuromedizin.

Die Aufgabe wird somit erfindungsgemäß gelöst durch die Verwendung eines Hydrogels, welches *α*-L-Guluronsäure (GulUA)- und/oder *β*-D-Mannuronsäure (ManUA)-haltige Polysaccharide oder deren Salze enthält, wobei die Kohlenhydrateinheiten nicht durch chemische Linker kovalent quervernetzt sind, zur Behandlung von Beschädigungen des Nervensystems, zur Förderung des Nervenwachstums bzw. Neuritenwachstums und/oder der Kultivierung von Neuronen und/oder zur Behandlung oder Prophylaxe von neurodegenerativen Erkrankungen. Erfindungsgemäß wird das Hydrogel in Gegenwart von zwei- oder mehrwertigen Kationen mit einer Konzentration von 1 bis 4 mmol/l, bevorzugt maximal 3 mmol/l, bevorzugt zwischen 1,5 und 3 mmol/l, besonders bevorzugt zwischen 1,6 und 2,5 mmol/l, insbesondere zwischen 1,8 und 2,2 mmol/l hergestellt. Die Konzentration an zwei- oder mehrwertigen Kationen beträgt vorzugsweise mindestens 1 µmol/l, bevorzugt mindestens 100 µmol/l, besonders bevorzugt mindestens 300 µmol/l.

Besonders überraschend war die Entdeckung der Erfinder, dass das Wachstum von Neuriten allein durch das erfindungsgemäß verwendete Hydrogel gefördert wird, ohne dass dieses weitere Zusätze enthält, die üblicherweise zur Förderung des Zellwachstums eingesetzt werden (beispielsweise Komponenten der extrazellulären Matrix oder Wachstumsfaktoren). Der wachstumsfördernde Effekt kann allein den erfindungsgemäß verwendeten Hydrogelen zugeschrieben werden. Bevorzugt enthält das erfindungsgemäß verwendete Hydrogel daher keine weiteren solchen Zusätze, insbesondere keine Komponenten der extrazellulären Matrix oder Wachstumsfaktoren.

Rheologische Untersuchungen von Alginathydrogelen mit unterschiedlichen Konzentrationen an multivalenten Kationen zeigten, dass Alginathydrogele mit Kationengehalten von unter 4 mmol/l charakteristische Quellungsseigenschaften zeigen, die bei Alginathydrogelen mit höheren Kationengehalten nicht beobachtet werden. Alginathydrogele mit Kationengehalten von unter 4 mmol/l zeigen eine nichtlinear zunehmende Quellung, je geringer die Kationengehalte sind. Nur für Alginathydrogele mit derart geringen Kationengehalten wurden die überraschenden wachstumsfördernden Eigenschaften auf das Neuritenwachstum festgestellt.

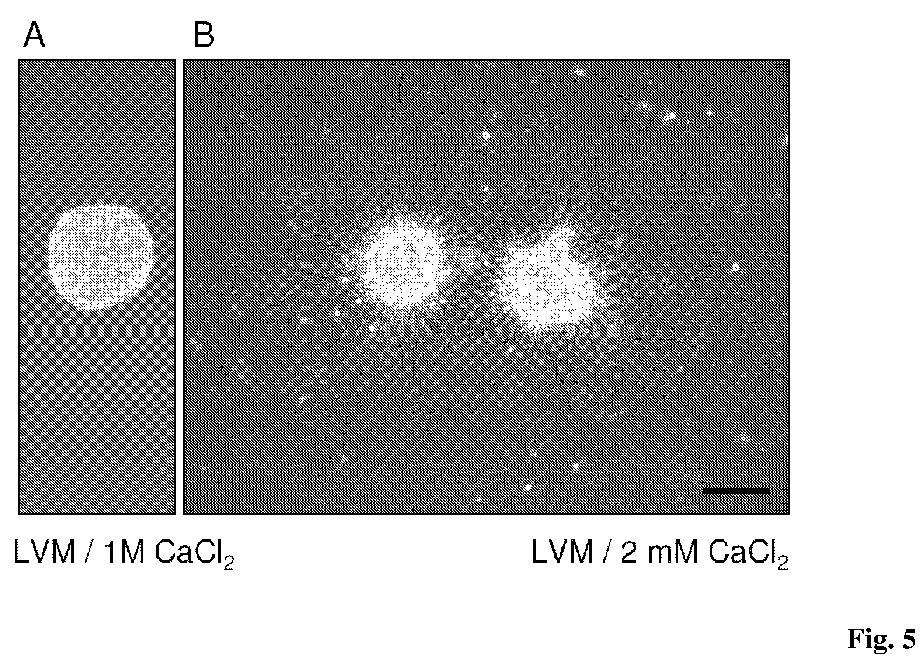

Zusätzlich konnten die Erfinder beobachten, dass die erfindungsgemäß verwendeten Hydrogele Nervenzellen vor oxidativem Stress schützen (siehe **Fig. 5**). Oxidativer Stress wird als eine wichtige Ursache für neurodegenerative Erkrankungen beschrieben. Die erfindungsgemäß verwendeten Polysaccharide können daher vorteilhaft auch prophylaktisch bei neurodegenerativen Erkrankungen eingesetzt werden, oder auch um Stresssituationen für Neuronen entgegenzuwirken, wie sie z. B. bei chirurgischen Eingriffen entstehen.

Das erfindungsgemäße Hydrogel enthält Polysaccharide, welche aus einzelnen Kohlenhydrateinheiten (Monosacchariden) von *α*-L-Guluronsäure (GulUA) und *β*-D-Mannuronsäure (ManUA) zusammengesetzt sind. Polysaccharide im Sinne der Erfindung sind makromolekulare Kohlenhydrate, welche aus mehr als 10, meistens deutlich mehr, bevorzugt mindestens 100, Kohlenhydrateinheiten bestehen. Die Polysaccharide werden allgemein auch als Glykane bezeichnet. In den bevorzugt linearen Polysacchariden sind die einzelnen Kohlenhydrateinheiten 1,4-glykosidisch miteinander verknüpft.

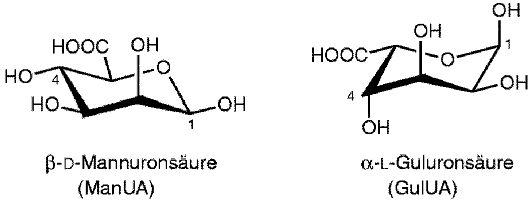

Bevorzugte lineare Polysaccharide, welche aus GulUA und ManUA zusammengesetzt sind, sind Alginsäuren, welche beispielsweise in der Zellwand von Algen vorkommen. Die Salze der Alginsäuren sind die Alginate. In Alginsäuren bzw. Alginaten sind die einzelnen Monosaccharideinheiten 1,4-glykosidisch miteinander verknüpft, wobei der Anteil an GulUA und ManUA variieren kann. Vorzugsweise werden unmodifizierte Alginsäuren oder Alginate zur Herstellung erfindungsgemäß verwendeter Hydrogele eingesetzt.

In Gegenwart von multivalenten Kationen bilden sich zwischen den funktionellen Gruppen der einzelnen Kohlenhydrateinheiten der Polysaccharide, insbesondere zwischen den Carboxylgruppen der GulUA-Einheiten und den Kationen ionische und somit nicht-kovalente Bindungen aus. Dadurch entstehen in Gegenwart von zwei- oder mehrwertigen Kationen Hydrogele. Unter einem erfindungsgemäßen Hydrogel versteht man ein dreidimensionales Polymernetzwerk eines hydrophilen Polysaccharides, welches als Quellungsmittel Wasser enthält, und welches im Fall von Alginat intermolekular einzig durch ionische bzw. elektrostatische Wechselwirkungen, jedoch nicht durch kovalente Bindungen verknüpft ist.

In Wasser oder wässrigen Lösungen quillt dieses Netzwerk unter weitgehender Formerhaltung bis zu einem Gleichgewichtsvolumen auf.

Hydrogele können auch gebildet werden, indem die funktionellen Gruppen der erfindungsgemäß verwendeten Polysaccharide durch chemische Linker intermolekular kovalent quervernetzt werden. In der vorliegenden Erfindung werden jedoch keine chemischen Linker verwendet, so dass die Kohlenhydrateinheiten nicht kovalent quervernetzt sind.

In der charakteristischen dreidimensionalen Struktur sind die zwei- oder mehrwertigen Kationen in die Glykanstruktur eingebettet. Da das Kation in dieser Struktur wie ein Ei in der Schachtel liegt wird das Modell dieser Struktur auch als "Eierschachtel-Modell" oder "egg-box-model" bezeichnet.

Die Ausbildung dreidimensionaler Netzwerke aus den Polysacchariden erfolgt in Gegenwart von zwei- oder mehrwertigen Kationen. Von den Erfindern wurde überraschend festgestellt, dass in Gegenwart von geringen Kationenkonzentrationen von maximal 4 mmol/l, bevorzugt zwischen 1 und 4 mmol/l, besonders bevorzugt zwischen 1,5 und 3 mmol/l, ganz besonders bevorzugt zwischen 1,6 und 2,5 mmol/l, das Neuritenwachstum durch die bei diesen Bedingungen gebildeten Alginat-Hydrogele gefördert wurde. Dies war insofern nicht zu erwarten, als bei den aus dem Stand der Technik bekannten Verwendungen von Alginaten (zur Einkapselung oder als Trägerstruktur) deutlich höhere Konzentrationen an zwei- oder mehrwertigen Kationen verwendet wurden, bei denen diese Effekte auf Nervenzellen nicht beobachtet wurden. Die Erfinder konnten nachweisen, dass der vorteilhafte Effekt auf das Wachstum von Neuriten bei Konzentrationen an zwei- oder mehrwertigen Kationen, die üblicherweise z. B. für die Herstellung von Kapillarhydrogelen verwendet werden (100 mmol/l und mehr), nicht auftritt (siehe **Fig. 6****).** Entsprechende Beobachtungen wurden in Bezug auf die Adhäsion von Neuronen auf Oberflächen, die mit dem beschriebenen Alginat-Hydrogel beschichtet worden waren, gemacht: auch hier wurde ein günstiger adhäsionsfördernder Effekt nur bei Konzentrationen der verwendeten zwei- oder mehrwertigen Kationen von 1 bis 4 mmol/l, bevorzugt zwischen 1,5 und 3 mmol/l, ganz besonders bevorzugt zwischen 1,6 und 2,5 mmol/l, beobachtet.

Vorzugsweise werden die erfindungsgemäß verwendeten Hydrogele daher in Gegenwart von Kationenkonzentrationen von 1 bis 4 mmol/l, bevorzugt zwischen 1,5 und 3 mmol/l, besonders bevorzugt zwischen 1,6 und 2,5 mmol/l hergestellt..

Durch die vorteilhaften Eigenschaften der Hydrogele auf das Wachstum von Neuriten und die Adhäsion von Neuronen auf Oberflächen eignen sie sich zur Verwendung in der Behandlung von Beschädigungen des Nervensystems, zur Förderung des Neuritenwachstums und/oder zur Behandlung oder Prophylaxe von neurodegenerativen Erkrankungen sowie für die Kultivierung von Neuronen in beliebigen Zellkulturgefäßen oder -reaktoren.

Die Festigkeit des Hydrogels erhöht sich mit der Konzentration der zur Herstellung eingesetzten zwei- oder mehrwertigen Kationen. Zur Herstellung der erfindungsgemäß verwendeten Hydrogele werden Kationenlösungen mit einer Konzentration eingesetzt, die maximal 4 mmol/l, vorzugsweise zwischen 1 und 4 mmol/l, bevorzugt zwischen 1,5 und 3 mmol/l, besonders bevorzugt zwischen 1,6 und 2,5 mmol/l beträgt. Aus dem Stand der Technik bekannte Alginathydrogele, welche durch Kombination mit Lösungen zwei- oder mehrwertiger Kationen mit Konzentrationen von 100 mmol/l und mehr hergestellt werden, besitzen eine festere Konsistenz. Die erfindungsgemäßen Hydrogele, welche durch Kombination mit Lösungen zwei- oder mehrwertiger Kationen mit Konzentrationen von 1 bis 4 mmol/l, besonders bevorzugt zwischen 1,5 und 3 mmol/l, ganz besonders bevorzugt zwischen 1,6 und 2,5 mmol/l, gebildet werden, sind zähflüssig bis geleeartig.

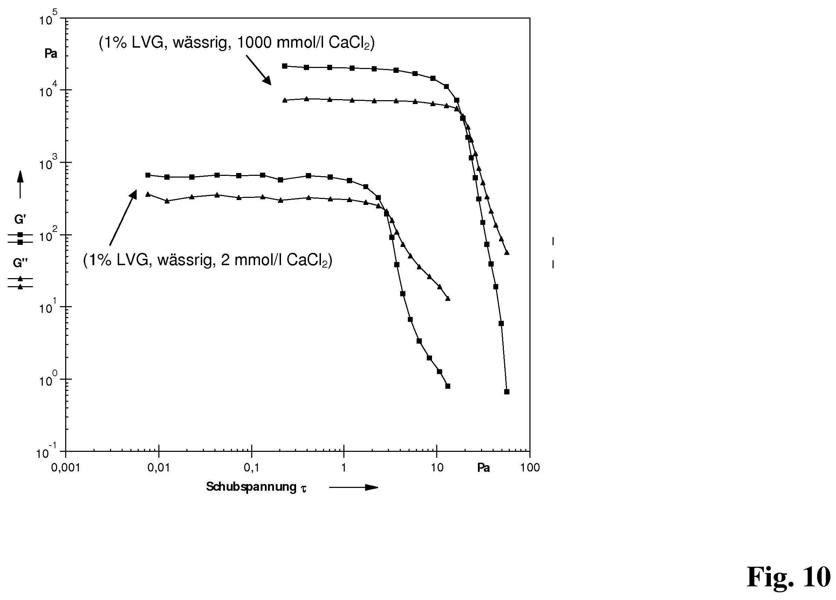

Experimentelle Untersuchungen der Erfinder konnten zeigen, dass Hydrogele, welche in Gegenwart von hohen Konzentrationen mehrwertiger Kationen hergestellt wurden, ein in Bezug auf die Viskosität ca. 30mal höheres Speichermodul besitzen, als Hydrogele, welche in Gegenwart von erfindungsgemäß eingesetzten Konzentrationen an mehrwertigen Kationen hergestellt wurden (siehe **Fig. 10**).

Erfindungsgemäß verwendete Hydrogele weisen ein Speichermodul von vorzugsweise weniger als 10 kPa, bevorzugt weniger als 5 kPa, weiter bevorzugt weniger als 2 kPa, besonders bevorzugt weniger als 1 kPa auf.

Außerdem weisen die erfindungsgemäßen Hydrogele keine makroskopisch sichtbare anisotrope Kapillarstruktur auf, wie bekannte nach dem Verfahren der gerichteten ionotropen Gelbildung bei hohen Kationenkonzentrationen (100 mmol/l und mehr) erzeugte feste Hydrogele.

Eigenschaften der Hydrogele können durch die Variation des Anteils der Monosaccharideinheiten von GulUA und ManUA und die Variation der Vernetzungsdichte, welche sich aus der Menge und der Art der eingesetzten Kationen ergibt, gezielt eingestellt werden.

Von den Erfindern wurde überraschend festgestellt, dass sich bei Verwendung von Calciumionen besonders Hydrogele mit einem hohen Anteil an ManUA dafür eignen, das Wachstum von Neuriten zu fördern **(****Fig. 3****).** Daher umfasst die Erfindung in einer bevorzugten Ausgestaltung die Verwendung eines Hydrogels, welches Polysaccharide enthält, welche mehr Kohlenhydrateinheiten von ManUA als von GulUA enthalten. Vorzugsweise ist das Verhältnis von GulUA und ManUA in diesen Polysacchariden ≤ 1. Besonders bevorzugt beträgt der Anteil an ManUA im Polysaccharid mehr als 50 %. Insbesondere wenn Zn²⁺ als zweiwertiges Kation (alleine oder in Kombination mit anderen) zur Hydrogelbildung eingesetzt wird, können jedoch auch Polysaccharide mit einem niedrigeren Anteil an ManUA oder ohne ManUA eingesetzt werden.

Die Ausbildung dreidimensionaler Netzwerke aus den Polysacchariden erfolgt in Gegenwart der erfindungsgemäß definierten Konzentration von zwei- oder mehrwertigen Kationen. Vorzugsweise enthält das Hydrogel zwei- oder dreiwertige Kationen, die bevorzugt ausgewählt sind aus Al³⁺, Ba²⁺, Ca²⁺, Cd²⁺, Cu²⁺, Fe²⁺, Fe³⁺, Mn²⁺, Ni²⁺, Pb²⁺, Sn²⁺, Sr²⁺ und Zn²⁺. Besonders bevorzugt sind die zwei- oder mehrwertigen Kationen ausgewählt aus Ca²⁺, Sr²⁺, Ba²⁺, Cu²⁺, Ni²⁺, Zn²⁺ und Fe³⁺. Ganz besonders bevorzugt sind die Kationen Calciumionen.

Zur Herstellung der Hydrogele werden wässrige Lösungen der o. a. Kationen verwendet. Dabei ist die Konzentration der Kationenlösung vorzugsweise maximal 5 mmol/l, bevorzugt maximal 4 mmol/l, weiter bevorzugt zwischen 1 und 4 mmol/l, besonders bevorzugt zwischen 1,5 und 3 mmol/l, ganz besonders bevorzugt zwischen 1,6 und 2,5 mmol/l. Die Konzentration der Kationenlösung beträgt vorzugsweise mindestens 1 µmol/l, bevorzugt mindestens 100 µmol/l, besonders bevorzugt mindestens 300 µmol/l.

Vorzugsweise enthalten die zur Herstellung der Hydrogele eingesetzten wässrigen Kationenlösungen mehrere verschiedene zwei- oder mehrwertige Kationen in den genannten Konzentrationen. Dies führt dazu, dass im gebildeten Hydrogel mehrere verschiedene zwei- oder mehrwertige Kationen durch ionische, elektrostatische bzw. nicht kovalente Bindungen zu mit den Polysaccharideinheiten zu einem dreidimensionalen Netzwerk verknüpft sind. Daher enthält ein erfindungsgemäß verwendetes Hydrogel bevorzugt unterschiedliche zwei- oder mehrwertige Kationen. Dabei beträgt die Gesamtkonzentration der zwei- oder mehrwertigen Kationen im Hydrogel bevorzugt maximal 4 mmol/l, bevorzugt zwischen 1 und 4 mmol/l, besonders bevorzugt zwischen 1,5 und 3 mmol/l, ganz besonders bevorzugt zwischen 1,6 und 2,5 mmol/l.

Bei der Bildung des Hydrogels werden nicht alle Kationen intermolekular in die Egg-Box-Struktur des Hydrogels eingebaut, so dass die Kationenkonzentration im Hydrogel von den o. a. Konzentrationen abweichen kann. Die Konzentration der Kationen im Hydrogel ist abhängig von den GluUA- und ManUA-Anteilen der Polysaccharide und variiert somit zwischen unterschiedlichen Polysaccharid-Typen und den verwendeten Aufreinigungsverfahren. Die Kationenkonzentration im Hydrogel bewegt sich jedoch in der Größenordnung (vorzugsweise ± 15 %, besonders bevorzugt ± 10 %) der Kationenkonzentration des gesamten Reaktionsvolumens (Kationenlösung + Polysaccharidsol).

Vorzugsweise beträgt die Konzentration der Polysaccharide im Polysaccharidsol, welches zur Herstellung des Hydrogels eingesetzt wird 0,1 bis 5 Gewichtsprozent, bevorzugt 0,2 bis 4 Gewichtsprozent, besonders bevorzugt 0,2 bis 2 Gewichtsprozent.

Bevorzugt sind die Polysaccharide nicht chemisch modifiziert, besonders bevorzugt unmodifzierte Alginate.

Die Herstellung erfindungsgemäß verwendeter Hydrogele erfolgt nach einer der nachfolgend beschriebenen Varianten:
a.) Zugabe von zwei- oder mehrwertigen Kationen (bevorzugt in wässriger Lösung) zu einem wässrigen Sol der GulUA- und/oder ManUA-haltigen Polysaccharide,
b.) Zugabe von zwei- oder mehrwertigen Kationen in wässriger Lösung zu den GulUA-und/oder ManUA-haltigen Polysacchariden in trockener Form,
c.) Zugabe von Wasser oder einer wäßrigen Lösung zu einem getrockneten Hydrogel, welches nach Variante a.) oder b.) hergestellt wurde und zwei- oder mehrwertige Kationen enthält,
d.) Zugabe von Wasser oder einer wässrigen Lösung zu einer Feststoffmischung aus einem GulUA- und/oder ManUA-haltigen Polysaccharid und einem Salz, welches zwei- oder mehrwertige Kationen enthält.

In Variante a.) wird eine wässrige Lösung der GulUA- und/oder ManUA-haltigen Polysaccharide oder deren Salze hergestellt. Zu dem wässrigen Polysaccharidsol wird eine wässrige Lösung gegeben, welche die zwei- oder mehrwertigen Kationen enthält. Der Gehalt an zwei- oder mehrwertigen Kationen in der resultierenden Mischung beträgt maximal 5 mmol/l, bevorzugt maximal 4 mmol/l, weiter bevorzugt zwischen 1 und 4 mmol/l, besonders bevorzugt zwischen 1,5 und 3 mmol/l, ganz besonders bevorzugt zwischen 1,6 und 2,5 mmol/l.

Alternativ wird in der ersten Variante eine wässrige Lösung, die zwei- oder mehrwertige Kationen enthält, vorgelegt und anschließend ein wässriges Sol der Polysaccharide oder deren Salze zugegeben, so dass ein Hydrogel gebildet wird. Vorzugsweise wird die Kationenlösung unter Vermeidung einer Vermischung vorsichtig über das Polysaccharidsol geschichtet, so dass sich im unteren Teil des Gefäßes eine homogene Hydrogelschicht ausbildet und in einer oberen Phase die Kationenlösung verbleibt. In diesem Fall besteht das Hydrogel bevorzugt aus einer einzigen zusammenhängenden Schicht.

In einer anderen Ausgestaltung der Erfindung werden Kationenlösung und Polysaccharidsol vermischt. Durch die Vermischung erfolgt die Hydrogelbildung an den Stellen, wo Polysaccharidsol und Kationenlösung in Kontakt geraten. Dadurch entsteht ein Hydrogel in Form einer Suspension, in welcher das Gel eine unregelmäßige Morphologie aufweist.

In Variante b.) wird bevorzugt ein wässriges Sol der Polysaccharide oder deren Salzen mit den Eigenschaften der ersten Variante zunächst getrocknet. Zum Trocknen eignen sich alle bekannten Trockenverfahren, wie beispielsweise die Trocknung in Trockenschränken, Lufttrocknung, Gefriertrocknung oder Kritisch-Punkt-Trocknung. Bevorzugt wird das Polysaccharidsol an der Luft getrocknet. Das getrocknete Polysaccharid kann vorteilhaft bei 4 °C oder Raumtemperatur und vor Feuchtigkeit geschützt über Monate bis Jahre gelagert werden. Vor der Verwendung wird eine wässrige Lösung an zwei- oder mehrwertigen Kationen zugegeben, wodurch, bevorzugt innerhalb von 1 bis 60 Minuten, ein Hydrogel gebildet wird. Alternativ wird das getrocknete Polysaccharid nach der Zugabe der Kationenlösung für mehrere Stunden, insbesondere mindestens 24 h, belassen, um das erfindungsgemäß verwendete Hydrogel bereitzustellen.

In Variante c.) wird zunächst ein Hydrogel nach einer der oben genannten Varianten (Variante a.) wird bevorzugt) hergestellt und anschließend getrocknet. Das getrocknete Hydrogel kann vorteilhaft bei 4 °C oder Raumtemperatur und vor Feuchtigkeit geschützt über Monate bis Jahre gelagert werden. Vor der Verwendung wird hier bevorzugt das Hydrogel durch Zugabe von Wasser oder einer wässrigen Lösung (vorzugsweise Zellkulturmedium) rekonstituiert, bevorzugt dauert die Rekonstitution 1 bis 60 Minuten. Dabei wird Wasser oder die wässrige Lösung vorsichtig über das getrocknete Hydrogel geschichtet oder das getrocknete Hydrogel vorsichtig in Wasser oder eine wässrige Lösung eingelegt. Alternativ wird das getrocknete Hydrogel nach der Zugabe der Kationenlösung für mehrere Stunden, insbesondere mindestens 24 h, belassen, um ein erfindungsgemäß verwendetes rehydratisiertes Hydrogel bereitzustellen.

In Variante d.) wird eine feste Mischung aus GulUA- und/oder ManUA-haltigen Polysacchariden oder deren Salze und einem Salz, welches zwei- oder mehrwertige Kationen enthält, hergestellt. Vor der Verwendung wird hier bevorzugt das Hydrogel durch Zugabe von Wasser oder einer wässrigen Lösung (z. B. Zellkulturmedium) hergestellt, wobei so viel Wasser zugegeben wird, dass die Konzentration der zwei- oder mehrwertigen Kationen in der entstehenden wäßrigen Mischung 10 mmol/l, bevorzugt 5 mmol/l, besonders bevorzugt 4 mmol/l, nicht übersteigt, insbesondere zwischen 1 und 4 mmol/l, bevorzugt zwischen 1,5 und 3 mmol/l, besonders bevorzugt zwischen 1,6 und 2,5 mmol/l liegt.

Vorteilhaft können sterile Sole der Polysaccharide (in wässriger Lösung oder getrockneter Form) und getrocknete Hydrogele, wenn sie vor Feuchtigkeit geschützt gelagert werden oder steril und feucht gelagerte Hydrogele, bei 4 °C oder Raumtemperatur über Monate bis Jahre stabil aufbewahrt werden.

Durch den Entzug des Wassers aus dem erfindungsgemäß verwendeten Hydrogel entsteht eine feste Substanz, welche nach Inkontaktbringen mit Wasser oder einer wässrigen Lösung (z. B. von Körperflüssigkeiten oder Zellkulturmedien) aufquillt und zu einem Hydrogel mit vergleichbarem Effekt auf das Neuritenwachstum wie vor dem Trocknungsvorgang rekonstituiert. Daher liegt das Hydrogel in einer bevorzugten Ausgestaltung der Erfindung in getrockneter Form vor. Zum Trocknen eignen sich hierbei alle bekannten Verfahren, wie beispielsweise die Trocknung in Trockenschränken, Lufttrocknung, Gefriertrocknung oder Kritisch-Punkt-Trocknung.

Die Polysaccharide oder deren Salze, die erfindungsgemäß verwendet werden, sind biologisch abbaubar, d. h. sie werden im Körper mit der Zeit abgebaut. Die eingesetzten Polysaccharide oder Salze werden vor der Verwendung durch bekannte Verfahren sterilisiert. Bevorzugt werden die Polysaccharide durch Autoklavieren oder durch Sterilfiltration sterilisiert. Die Sterilisation durch Autoklavieren führt zur teilweisen Spaltung der Polysaccharidketten in kleinere Polysaccharideinheiten. Es konnte jedoch durch die Erfinder gezeigt werden, dass dies keinen negativen Einfluss auf den wachstumsfördernden Effekt hat (siehe **Fig. 3C**)**.**

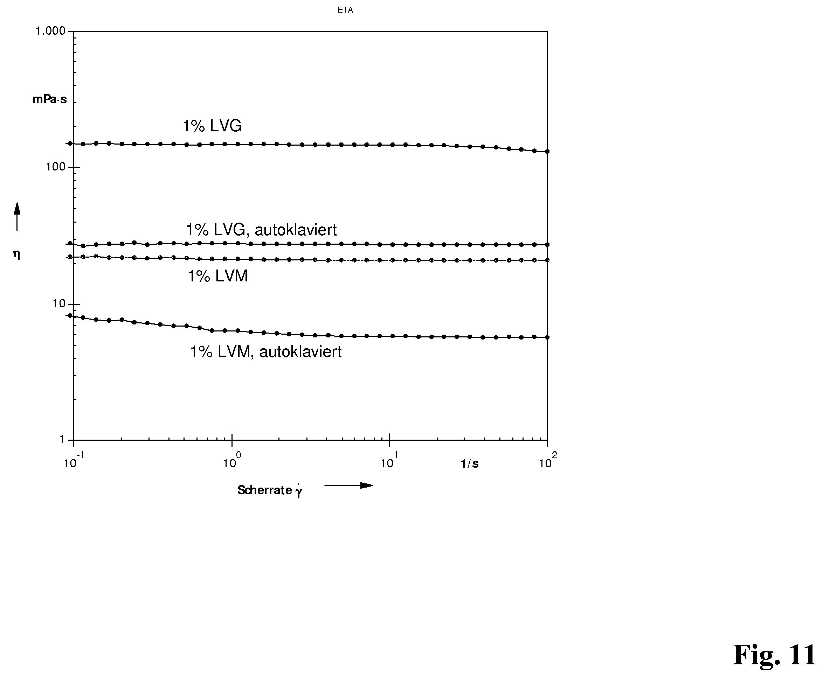

Durch das Autoklavieren und die damit verbundene Verkürzung der Polysaccharidketten besitzt das Polysaccharidsol nach dem Autoklavieren eine reduzierte Viskosität. Dies wurde durch experimentelle Vergleichsversuche der Erfinder bestätigt (siehe **Fig. 11**). Hierbei wurden je nach Alginat-Typ nach dem Autoklavieren Viskositätswerte gemessen, welche zwischen ca. 20 und 30 % derjenigen der Ausgangssole lagen.

Erfindungsgemäß verwendete Hydrogele liegen aufgrund deren Herstellung bevorzugt als homogenes Gemisch, also einer einzigen zusammenhängenden Schicht, oder in der Form einer Suspension vor. Sie eignen sich zur Implantation in den Körper, wobei dazu ein erfindungsgemäß verwendetes Hydrogel vorzugsweise in eine Defektstelle von zentralen Nervenbahnen sowie von peripheren Nerven implantiert wird. Die Erfindung umfasst daher auch ein Verfahren zur Implantation eines erfindungsgemäß verwendeten Hydrogels in den menschlichen Körper, vorzugsweise in eine Defektstelle von zentralen Nervenbahnen oder von peripheren Nerven. Nach der Implantation wachsen Neuriten der in Hydrogelnähe befindlichen Neuronen in das erfindungsgemäß verwendete Hydrogel ein. Das erfindungsgemäß verwendete Hydrogel fördert dabei aktiv das Wachstum der Neuriten von Nervenzellen.

Das Hydrogel liegt vorzugsweise zellfrei vor. Zellen werden entweder erst vom Endanwender zugegeben bzw. kommen bevorzugt erst *in vivo* (d. h. im Körper) mit diesen in Kontakt. Die Zugabe der Zellen (z. B. in Suspension) oder der Gewebekultur erfolgt dabei bevorzugt unter Vermeidung einer Vermischung zwischen Hydrogel und Zellen, so dass sich die Zellen, Zelllinien oder Gewebe auf dem erfindungsgemäß verwendeten Hydrogel ablagern. Dadurch wachsen die Zellen in zweidimensionaler Kultur auf der Oberfläche des erfindungsgemäß verwendeten Hydrogels. Durch die vorteilhaften Effekte des erfindungsgemäß verwendeten Hydrogels wird die Ausbildung und das Wachstum von Neuriten gefördert, welche sich auf dem Hydrogel ausbreiten und in dieses einwachsen können.

Die Erfindung umfasst auch eine pharmazeutische Zusammensetzung oder ein Implantatmaterial, welche(s) ein erfindungsgemäßes Hydrogel aus GulUA- und ManUA-haltigen Polysacchariden oder deren Salzen und eine oder mehrere pharmazeutisch akzeptable Hilfssubstanzen enthält. Vorteilhaft eignet sich die pharmazeutische Zusammensetzung zur Behandlung der o. a. Beschädigungen des Nervensystems, zur Behandlung oder Prophylaxe von Nervenschädigungen bei chirurgischen Eingriffen und zur Behandlung oder Prophylaxe von neurodegenerativen Erkrankungen.

Die pharmazeutische Zusammensetzung enthält bevorzugt pharmazeutisch akzeptable Hilfssubstanzen, wie etwa diejenigen, die erforderlich sind um näherungsweise physiologische Bedingungen zu ergeben und/oder die Stabilität oder Lagerungsfähigkeit des Hydrogels zu erhöhen. Bevorzugt enthält die pharmazeutische Zusammensetzung zusätzliche pharmakologische Wirkstoffe.

Ein erfindungsgemäßes Implantatmaterial enthält bevorzugt neben dem erfindungsgemäßen Hydrogel zusätzlich Träger- und/oder Stützmaterialien. Die Träger und/oder Stützmaterialien sind bevorzugt poröse Scaffolds, röhren-, schlauch- oder faser-förmige Strukturen, Vliese, Schäume, Gitter oder durch Rapid Prototyping-Verfahren erzeugte dreidimensionale Strukturen. In einer Alternative umfasst das erfindungsgemäße Implantatmaterial vor der Implantation eine wässrige Lösung GulUA- und ManUA-haltiger Polysaccharide oder es ist mit einem getrockneten Film überzogen, welcher GulUA- und ManUA-haltige Polysaccharide enthält. Nach der Implantation wird das erfindungsgemäß verwendete Hydrogel vor Ort durch die in der Körperflüssigkeit enthaltenen physiologischen Kationenkonzentrationen ausgebildet. Die Verwendung der Implantatmaterialien, die mit GulUA- und ManUA-haltigen Polysacchariden überzogen sind, ist ebenfalls von der Erfindung umfasst.

Des Weiteren umfasst die Erfindung auch einen pharmazeutischen Kit zur vereinfachten Herstellung eines erfindungsgemäß verwendeten Hydrogels. Dabei enthält das Kit
a) ein vorzugsweise in getrockneter Form vorliegendes erfindungsgemäß verwendetes Hydrogel und
b) eine wässrige Flüssigkeit, welche einen Maximalgehalt an zwei- oder mehrwertigen Kationen von maximal 4 mmol/l enthält.

Der pharmazeutische Kit ermöglicht vorteilhaft eine schnelle und sterile Herstellung der erfindungsgemäß verwendeten Hydrogele. Dazu liegen die Polysaccharide a) vorzugsweise in getrockneter Form vor und werden mit der Flüssigkeit b) vor der Verwendung vermischt oder benetzt. Dadurch wird eine lange Lagerfähigkeit des Produkts ermöglicht.

Im pharmazeutischen Kit liegt das Hydrogel vorzugsweise zellfrei vor. Zellen werden entweder erst vom Endanwender zugegeben bzw. kommen bevorzugt erst *in vivo* (d. h. im Körper) mit diesen in Kontakt.

Vorzugsweise enthält die Flüssigkeit b) einen Gehalt an zwei- oder mehrwertigen Kationen von zwischen 1,5 und 3 mmol/l, besonders bevorzugt zwischen 1,6 und 2,5 mmol/l, insbesondere 1,8 und 2,2 mmol/l, vorzugsweise 2,0 mmol/l.

Die GulUA- und/oder ManUA-haltigen Polysaccharide sind bevorzugt wie oben genannt ausgewählt und können auch in Form eines getrockneten Hydrogels vorliegen (welches zwei- oder mehrwertige Kationen enthält). In dieser Variante ist die wässrige Flüssigkeit bevorzugt Wasser oder eine physiologische Kochsalzlösung. In einer weiteren Variante liegt eine Mischung aus den Polysacchariden und Metallsalzen, welche zwei- oder mehrwertige Kationen enthalten, in fester Form vor. Durch Vereinigung mit Wasser oder einer wässrigen Lösung und kontinuierlicher Vermischung wird das beschriebene Hydrogel *in situ* erzeugt. Die erfindungsgemäße pharmazeutische Zusammensetzung, das Implantatmaterial und der pharmazeutische Kit werden bevorzugt zur Behandlung oder Prophylaxe von Beschädigungen des Nervensystems, zur Förderung des Nervenwachstums, zur Behandlung oder Prophylaxe von Nervenschädigungen bei chirurgischen Eingriffen und/oder zur Behandlung oder Prophylaxe von neurodegenerativen Erkrankungen verwendet.

Bevorzugt wird das Hydrogel, die pharmazeutische Zusammensetzung, das Implantatmaterial oder der Kit zur Behandlung von Schlaganfall, insbesondere ischaemischem Schlaganfall, Schädel-Hirn-Trauma, Multipler Sklerose, Akuter disseminierten Enzephalomyelitis, Amyotropher Lateralsklerose (ALS), Retinopathia pigmentosa, Leichten kognitiven Beeinträchtigungen, Alzheimer, Pick-Krankheit, Altersdemenz, Progressiver supranukleären Blickparese, Subkortikaler Demenzen, Morbus Wilson, Multi-Infarkt-Demenz, arteriosklerotischer Demenz, AIDS-assoziierter Demenz, Zerebraler Degeneration, spinozerebellaerer Degeneration, Friedreich-Ataxie, Louis-Bar-Syndrom, Epilepsie-assoziierten Hirnschäden, Rückenmarksschäden, Restless-Legs-Syndrom, Chorea Huntington, Morbus Parkinson, Multisystematrophie, Zerebraler Vaskulitis, Mitochondrialen Enzephalomyopathien, Neuronalen Ceroid-Lipofuszinosen, Spinaler Muskelatrophie, Lysosomalen Speicherkrankheiten mit ZNS-Beteiligung, Leukodystrophien, Harnstoffzyklusdefekten, Hepatischer Enzephalopathie, Renaler Enzephalopathie, metabolischen Enzephalopathien, Porphyrie, bakterieller oder viraler Meningitis oder Meningoenzephalitis, Prionenerkrankungen, Vergiftungen mit neurotoxischen Verbindungen, Guillain-Barre-Syndrom, chronisch inflammatorischen Neuropathien, Polymyositis, Dermatomyositis, oder Strahlungs-induzierten Hirnschädigungen verwendet.

Für therapeutische Anwendungen wird einem Patienten eine sterile pharmazeutische Zusammensetzung oder ein erfindungsgemäß verwendetes Hydrogel verabreicht, um vorstehend beschriebene Erkrankungen zu behandeln.

Die Erfindung eignet sich zur Anwendung in einem Verfahren zur Behandlung von Beschädigungen des Nervensystems, zur Förderung des Nervenwachstums, zur Behandlung oder Prophylaxe von Nervenschädigungen bei chirurgischen Eingriffen und/oder zur Behandlung oder Prophylaxe von neurodegenerativen Erkrankungen. In dem Verfahren werden einem Patienten ein erfindungsgemäß verwendetes Hydrogel oder eine erfindungsgemäße pharmazeutische Zusammensetzung in oder um periphere Nerven implantiert. Durch die Implantation des Hydrogels werden das Wachstum der Nervenzellen und/oder das Auswachsen von Neuriten aus denselben angeregt. Das erfindungsgemäß verwendete Hydrogel ist biokompatibel. Da das Hydrogel biologisch abbaubar ist, wird dieses im Körper mit der Zeit komplett abgebaut. Insbesondere werden mit dem erfindungsgemäßen Verfahren neurodegenerative Erkrankungen oder Beschädigungen des Nervensystems behandelt, die aus den oben genannten Erkrankungen ausgewählt sind.

Zur Behandlung von Rückenmarksschäden umfasst das Verfahren die Implantation eines erfindungsgemäß verwendeten Hydrogels, einer erfindungsgemäßen pharmazeutischen Zusammensetzung oder eines erfindungsgemäßen Implantatmaterials in das Rückenmark eines Patienten. Dort wird das Wachstum von Neuriten angeregt und somit die Regeneration einer Verbindung von Nervenzellen bei geschädigtem Rückenmark ermöglicht.

Bei Erkrankungen, die mit Nervenschädigungen im Gehirn zusammenhängen, wird ein erfindungsgemäß verwendetes Hydrogel, eine erfindungsgemäße pharmazeutische Zusammensetzung oder ein erfindungsgemäßes Implantatmaterial in das Gehirn eines Patienten implantiert, um das Wachstum von Neuriten anzuregen.

Vorzugsweise wird das erfindungsgemäß verwendete Hydrogel zur Förderung der Regeneration von zentralen oder peripheren Neuronen implantiert. Besonders bevorzugt wird das Hydrogel, die pharmazeutische Zusammensetzung oder das erfindungsgemäße Implantatmaterial in oder um periphere Nerven oder in das Rückenmark oder in das Gehirn implantiert, um das Neuritenwachstum zu fördern.

Aufgrund der vorteilhaften Eigenschaften der erfindungsgemäß verwendeten Hydrogele mit einem Maximalgehalt von zwei- oder mehrwertigen Kationen von 1 bis 4 mmol/l, bevorzugt zwischen 1,5 und 3 mmol/l, ganz besonders bevorzugt zwischen 1,6 und 2,5 mmol/l, insbesondere 1,8 und 2,2 mmol/l, vorzugsweise 2,0 mmol/l, auf das Wachstum von Neuriten eignen sich diese zur *in vitro*-Kultivierung von Nervenzellen.

Daher umfasst die Erfindung auch die Verwendung eines Zellkulturbehälter oder Trägermaterials, welche mit erfindungsgemäßen Hydrogel beschichtet sind, für die Kultivierung von Zellen des Nervensystems (einschließlich Zelllinien) und/oder Geweben des Nervensystems.. Die Beschichtung erfolgt bevorzugt durch die Zugabe eines sterilen wässrigen Sols des Polysaccharides oder Polysaccharidsalzes und der anschließenden Trocknung. Dadurch verbleibt auf der Oberfläche des Zellkulturbehälters oder Trägermaterials eine Schicht des Polysaccharids, welches nach Zugabe einer wässrigen Lösung, welche einen Maximalgehalt von zwei- oder mehrwertigen Kationen von 4 mmol/l aufweist, insbesondere 2 mmol/l, bevorzugt innerhalb von 1 bis 60 Minuten ein Hydrogel bildet. Bevorzugt wird als wässrige Lösung zur Bildung des Hydrogels ein nach dem Stand der Technik bekanntes Zellkulturmedium eingesetzt, welches die bevorzugten Konzentrationen an di- oder multivalenten Kationen aufweist. Weiter bevorzugte Konzentrationen an zwei- oder mehrwertigen Kationen sind zwischen 1,5 und 3 mmol/l, ganz besonders bevorzugt zwischen 1,6 und 2,5 mmol/l. Erfindungsgemäß verwendete Zellkulturbehälter sind alle nach dem Stand der Technik bekannten Flaschen, Schalen, Platten oder Reaktoren, mit denen *in vitro* Zellen oder auch Gewebe kultiviert werden können. Bevorzugt sind die Zellkulturbehälter aus Kunststoff. Erfindungsgemäß verwendete Trägermaterialien sind alle Materialien, die zur Zellkultur als Träger eingesetzt werden, und jene Materialien, die als Träger für Zellen eingesetzt werden und die sich für die Implantation in den menschlichen Körper eignen.

Zu erfindungsgemäß verwendeten Trägermaterialien zählen Objektträger, welche mit den entsprechenden Zellen besiedelt werden können, oder dreidimensionale Körper, insbesondere für die Zellkultur geeignete Scaffolds, vorzugsweise Scaffolds aus porösen Materialien, die als Stützstrukturen in der Zellkultur eingesetzt werden.

In der Anwendung wird ein erfindungsgemäß verwendeter Zellkulturbehälter oder ein erfindungsgemäß verwendetes Trägermaterial für die Zellkultur bereitgestellt und anschließend mit einer Zellsuspension, welche isolierte Zellen oder Zelllinien oder auch Gewebe des Nervensystems in geeignetem Zellkulturmedium enthält, überschichtet. Ist ein erfindungsgemäß verwendeter Zellkulturbehälter mit einem erfindungsgemäß verwendeten Hydrogel beschichtet, liegt das Hydrogel vorzugsweise in getrockneter Form vor und wird durch die Zugabe einer wässrigen Flüssigkeit (Flüssigkeit (b) wie oben definiert: Zellkulturmedium oder der Zellsuspension) rehydratisiert. Erfindungsgemäß verwendete Zellkulturbehälter, welche mit einem getrockneten Hydrogel beschichtet sind, sind besonders bevorzugt. Diese sind vorteilhaft lange haltbar, insbesondere, wenn das Hydrogel vor dem Aufbringen auf die Oberfläche des Zellkulturbehälters oder Trägermaterials für die Zellkultur sterilisiert wurde. Weiterhin sind die erfindungsgemäß verwendeten Zellkulturbehälter einfach in der Handhabung. Besonders bevorzugt ist auf der Oberfläche des Zellkulturbehälters oder Trägermaterials für die Zellkultur eine einzige zusammenhängende Schicht des erfindungsgemäß verwendeten Hydrogels aufgebracht.

Die Zugabe der Zellen (z. B. in Suspension) oder der Gewebekultur erfolgt dabei bevorzugt unter Vermeidung einer Vermischung zwischen Hydrogel und Zellen, so dass sich die Zellen, Zelllinien oder Gewebe auf dem erfindungsgemäß verwendeten Hydrogel ablagern. Dadurch wachsen die Zellen in zweidimensionaler Kultur auf der Oberfläche des erfindungsgemäß verwendeten Hydrogels. Durch die vorteilhaften Effekte des erfindungsgemäß verwendeten Hydrogels wird die Ausbildung und das Wachstum von Neuriten gefördert, welche sich auf dem Hydrogel ausbreiten und in dieses einwachsen können.

Die so beschichteten Zellkulturbehälter oder Trägermaterialien werden erfindungsgemäß für die Kultivierung von Zellen (einschließlich Zelllinien) und/oder Geweben des Nervensystems verwendet. Für die *in vitro*-Kultivierung werden die Zellen, Zelllinien und/oder die Gewebe in bekannten Medien für die Zellkultur, insbesondere in Medien, welche für die Kultivierung von Nervenzellen geeignet sind, aufgenommen. Diese enthalten die erfindungsgemäßen Kationenkonzentrationen. Nach Zugabe des Mediums in die polysaccharid-beschichteten Zellkulturbehälter oder Trägermaterialien wird ein Hydrogel gebildet, welches vorteilhaft die Adhäsion von Neuronen und das Wachstum von Neuriten unterstützt.

Die Erfindung gibt eine Verwendung eines Hydrogels an, die es ermöglicht, die Bildung und das Wachstum von Neuriten von Nervenzellen zu fördern, ohne dass dazu ein Zusatz von Wachstumsfaktoren oder Komponenten der extrazellulären Matrix notwendig ist. Die erfindungsgemäß verwendeten Hydrogele führen mit unterschiedlichsten Zusammensetzungen zum Erfolg, Voraussetzung ist die Anwesenheit GulUA- und ManUA-haltiger Polysaccharide in wässriger Lösung mit einem Gesamtgehalt an multivalenten Kationen zwischen 1 mmol/l und 4 mmol/l. Die erfindungsgemäßen Hydrogele sind in der Anwendung äußerst einfach handhabbar, sie können problemlos getrocknet und wieder rehydratisiert werden. Auch eine Sterilisation des Hydrogels ist ohne Beeinträchtigung des wachstumsfördernden Effekts auf Neuriten möglich. Dadurch sind die erfindungsgemäß verwendeten Hydrogele für die *in vivo* Anwendung optimal geeignet. Da die Bestandteile des Hydrogels biologisch abbaubar und pharmakologisch unbedenklich sind, verbleiben nach Implantation und Abbau des erfindungsgemäß verwendeten Hydrogels im Körper keine Rückstände. Nebenwirkungen, die auf Rückständen eines derart ausgestalteten Implantatmaterials beruhen, sind daher nicht zu erwarten. Der wachstumsfördernde Effekt auf das Neuritenwachstum wurde sowohl *in vitro* als auch *in vivo* nach Implantation nachgewiesen. Es wurde nachgewiesen, dass die Implantation eines erfindungsgemäß verwendeten Hydrogels die Narbenbildung in Defektnähe vorteilhaft vermeidet. Sowohl die Ausbildung von glialen Narben als auch die Ausbildung von Narbengewebe nicht-neuronalen Ursprungs wird durch ein erfindungsgemäß verwendetes Hydrogel nach der Implantation vorteilhaft inhibiert.

Ein weiterer Vorteil der erfindungsgemäß verwendeten Hydrogele ist, dass Nervenzellen in Gegenwart der Hydrogele vor oxidativem Stress geschützt werden. Da oxidativer Stress ein Faktor ist, der mit der Entstehung von neurodegenerativen Erkrankungen in Verbindung gebracht wird, wirkt das Hydrogel vorteilhaft auch prophylaktisch gegenüber Schädigungen der Nervenzellen.

Durch die förderlichen Eigenschaften auf das Neuritenwachstum ist mit dem erfindungsgemäß verwendeten Hydrogel eine Möglichkeit gegeben, *in vitro* die Ausbildung von Neuriten zu fördern und geeignete Kulturbedingungen für die Kultivierung von Neuronen zu schaffen. Neuronen bilden in Zellkulturen ohne adhäsive Oberflächen sogenannte Sphäroide aus, deren Kultivierung nicht trivial ist. Eine Adhäsion der Sphäroide, unter der dann eine Neuritenbildung erfolgt, gelang bisher nur unzureichend. Durch die Beschichtung von handelsüblichen Zellkulturträgern mit einer Schicht aus dem erfindungsgemäß verwendeten Hydrogel wird bei Kultivierung die Adhäsion auch von Neuronen-Sphäroiden gefördert und die Neuritenbildung begünstigt.

Anhand folgender Figuren und Ausführungsbeispiele soll die Erfindung näher erläutert werden, ohne die Erfindung auf diese zu beschränken.

In den Beschreibungen der Figuren und Ausführungsbeispiele werden folgende Nomenklaturen für die nach unterschiedlichen Varianten hergestellten Alginat-Hydrogele verwendet. Allgemein werden die Hydrogele durch die allgemeine Formel ("x" % "Alginat" / "y" mol/l bzw. mmol/l "Salz") beschrieben. Dabei entspricht "x" die Konzentration des Alginatsols in Gewichts-Prozent, "Alginat" dem Typ des verwendeten Alginats (guluronsäurereiches LVG, mannuronsäurereiches LVM, SLM20, SLM100, alle vorgenannten Alginate sind kommerziell erhältliche Alginate der Firma NovaMatrix, gesondert gekennzeichnet sind Alginate anderer Hersteller, z.B. Sigma Aldrich), "y" der Konzentration der di- oder multivalenten Kationen, und "Salz" das eingesetzte Salz, das die di- oder multivalenten Kationen enthält. Durch den Zusatz "wässrig" werden Hydrogele beschrieben, die nach obenstehender Variante a.) (flüssiges Alginatsol wird mit flüssiger Kationenlösung versetzt) hergestellt wurden. Durch den Zusatz "rehydratisiert" werden Hydrogele beschrieben, welche nach obenstehender Variante b.) (Trocknung eines flüssigen Alginatsols, danach Versetzen mit flüssiger Kationenlösung) hergestellt wurden.

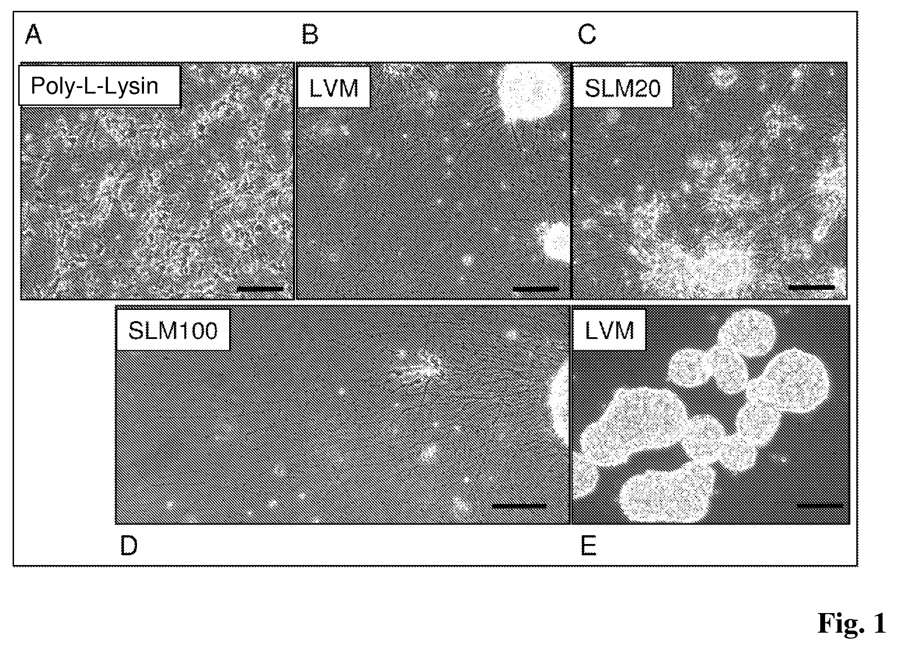

- Fig. 1: zeigt Aufnahmen einer Mikroskop-Analyse von isolierten Nervenzellen, welche unter verschiedenen Zellkulturbedingungen kultiviert wurden (die Skala entspricht 100 µm). Die Abbildungen (A) und (E) sind Vergleichsbeispiele. Die Nervenzellen wurden in einer Dichte von 1x10⁶ Zellen je 9,6 cm² für 72 h (A) auf Poly-L-lysin-beschichteten Plastikplatten, (B-D) auf einem (0,2% Alginat, wässrig / 1,8 mmol/l CaCl₂)-Hydrogel und (E) auf einem (0,2% Alginat, wässrig und 1 mol/l CaCl₂)-Hydrogel kultiviert. Es wurden mannuronsäurereiche Alginate der Typen (B, E) LVM, (C) SLM20 und (D) SLM100 verwendet.

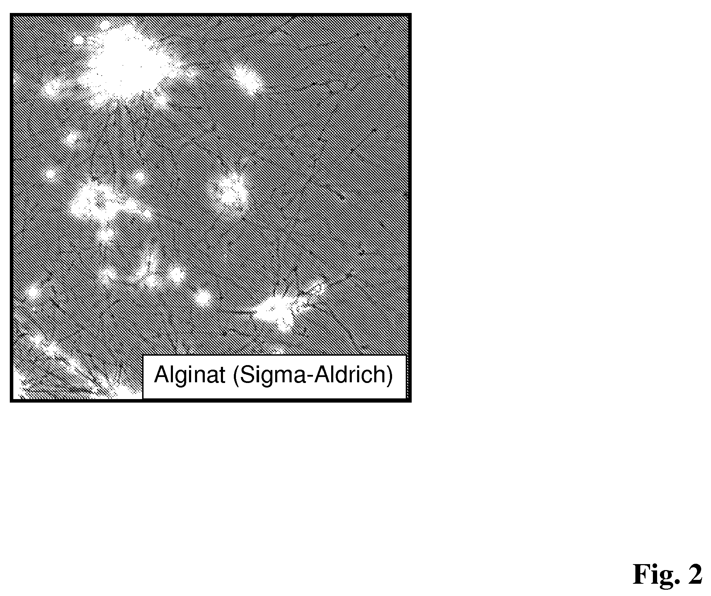

- Fig. 2: zeigt Aufnahmen einer Mikroskop-Analyse von isolierten Nervenzellen, welche unter verschiedenen Zellkulturbedingungen kultiviert wurden (die Skala entspricht 100 µm). Die Nervenzellen wurden in einer Dichte von 1x10⁶ Zellen je 9,6 cm² für 72 h auf einem (2% Alginat, wässrig / 1,8 mmol/l CaCl₂)-Hydrogel kultiviert. Es wurde Natriumalginat der Firma Sigma-Aldrich verwendet.
- Fig. 3: zeigt Aufnahmen einer Mikroskop-Analyse von frisch aus Rattenhirnen isolierten Nervenzellen, welche in einer Dichte von 1x10⁶ Zellen je 9,6 cm² für 48 h (A) auf Poly-L-Lysin-beschichteten Plastikplatten (Vergleichsbeispiel) und auf einem (1 % LVM, rehydratisiert / 1,8 mmol/l CaCl₂)-Hydrogel, wobei das Alginatsol (B) sterilfiltriert und (C) autoklaviert wurde, kultiviert wurden. Die Skala entspricht 100 µm. In (D) ist der Vergleich des Neuritenwachstums bei Verwendung von mannuronsäurereichem LVM und guluronsäurereichem LVG dargestellt. Dafür wurde die Anzahl der Neuriten je Bild (10x Vergrößerung) von mindestens 10 Bildern je Alginat nach 24 h und 48 h in Kultur statistisch ausgewertet. Die beobachteten Unterschiede waren statistisch signifikant (p < 0,05); hierzu wurde ein t-Test nach Student verwendet.

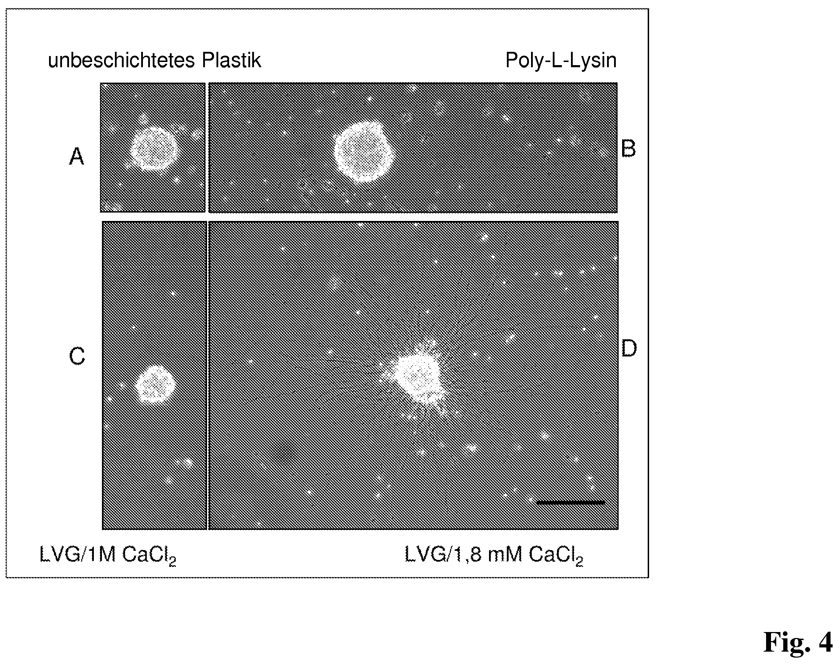

- Fig. 4: zeigt Aufnahmen einer Mikroskop-Analyse von frisch aus Rattenhirnen isolierten, zu Sphäroiden reassemblierten Nervenzellen nach Kultivierung auf (A) unbeschichteten, (B) mit Poly-L-Lysin beschichteten Zellkulturplatten (aus Kunststoff), (C) einem (0,2% LVG, wässrig / 1 mol/l CaCl₂)-Hydrogel (A - C sind Vergleichsbeispiele) und (D) einem (0,2 % LVG, wässrig / 1,8 mmol/l CaCl₂)-Hydrogel. Die Skala entspricht 100 µm.
- Fig. 5: zeigt Aufnahmen einer Mikroskop-Analyse von frisch aus Rattenhirnen isolierten, zu Sphäroiden reassemblierten Nervenzellen nach Kultivierung auf (A) einem (0,2% LVM, wässrig / 1 mol/l CaCl₂)-Hydrogel (Vergleichsbeispiel) und (B) einem (0,2% LVM, wässrig / 1,8 mmol/l CaCl₂)-Hydrogel. Die Skala entspricht 100 µm.
- Fig. 6: zeigt die Freisetzung von Lactatdehydrognase (LDH) aus kortikalen Nervenzellen der Ratte nach 8 (links) und 14 (rechts) Tagen in der Zellkultur. Zur Induktion von oxidativem Stress wurde das Zellkulturmedium an den indizierten Zeitpunkten mit 100 µmol/1 H₂O₂ (schraffierte Balken) bzw. 200 µmol/L H₂O₂ (schwarze Balken) versetzt und die LDH-Freisetzung nach 15 h bestimmt. Im Kontrollversuch wurde kein H₂O₂ zugesetzt (weiße Balken). Die Nervenzellen wurden unter verschiedenen Kulturbedingen kultiviert, entweder auf Poly-L-Lysin-beschichteten Platten (PLL, als Vergleichsbeispiel), auf Poly-L-Lysin-beschichteten Platten in Gegenwart von 0,1% LVM, welches am 7. Kulturtag zugegeben wurde (PLL+LVM) und auf einem (0,2% LVM, rehydratisiert / 1,8 mmol/l CaCl₂)-Hydrogel. Die Werte wurden statistisch mit einem t-Test nach Student ausgewertet und zeigten signifikante Unterschiede (p < 0,05).

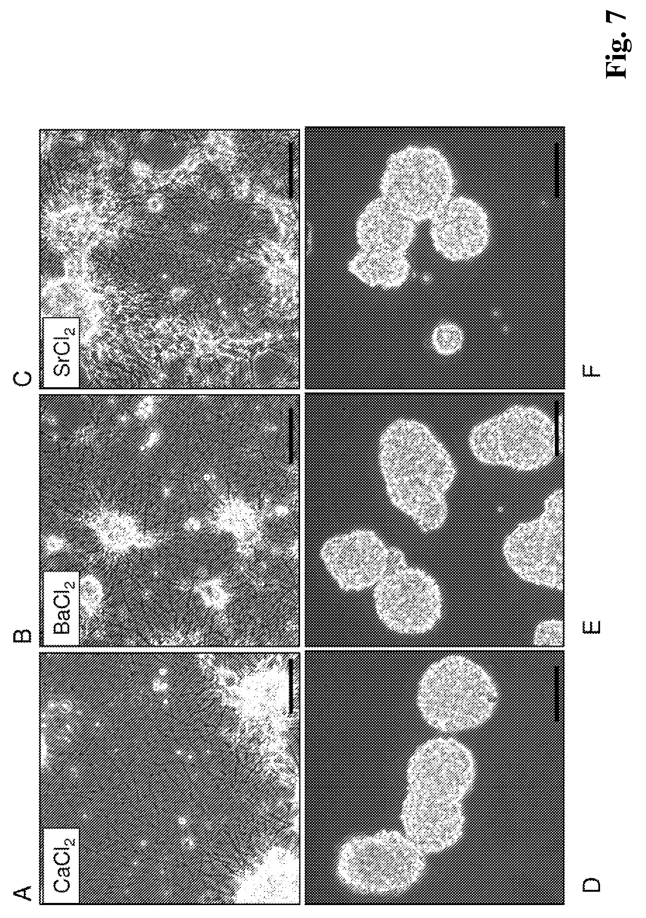

- Fig. 7: zeigt Aufnahmen einer Mikroskop-Analyse von Nervenzellen, welche auf (1% LVM, wässrig / 2 mmol/l Salz)-Hydrogelen (obere Reihe) oder (1% LVM, wässrig / 100 mmol/l Salz)-Hydrogelen (untere Reihe, als Vergleichsbeispiele) kultiviert wurden, wobei als Salz in (A, D) CaCl₂, (B, E) BaCl₂ und (C, F) SrCl₂ eingesetzt wurde. Die Skala entspricht 100 µm.

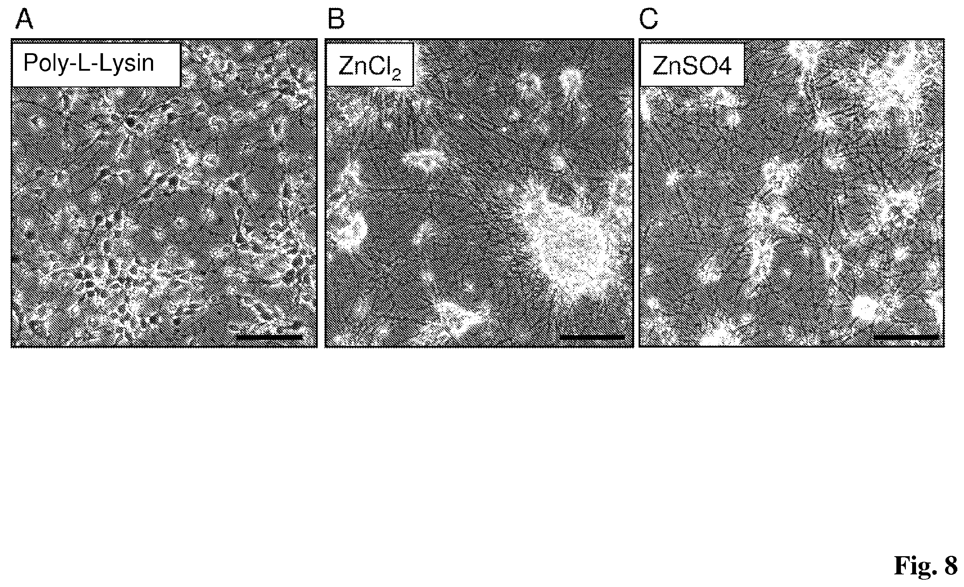

- Fig. 8: zeigt Aufnahmen einer Mikroskop-Analyse von Nervenzellen, welche auf mit Poly-L-Lysin beschichteten Platten (A, als Vergleichsbeispiel) und auf (1% LVG, wässrig / 2 mmol/l Salz)-Hydrogelen, wobei als Salz (B) ZnCl₂ und (C) ZnSO₄ verwendet wurde, kultiviert wurden. Die Skala entspricht 100 µm.

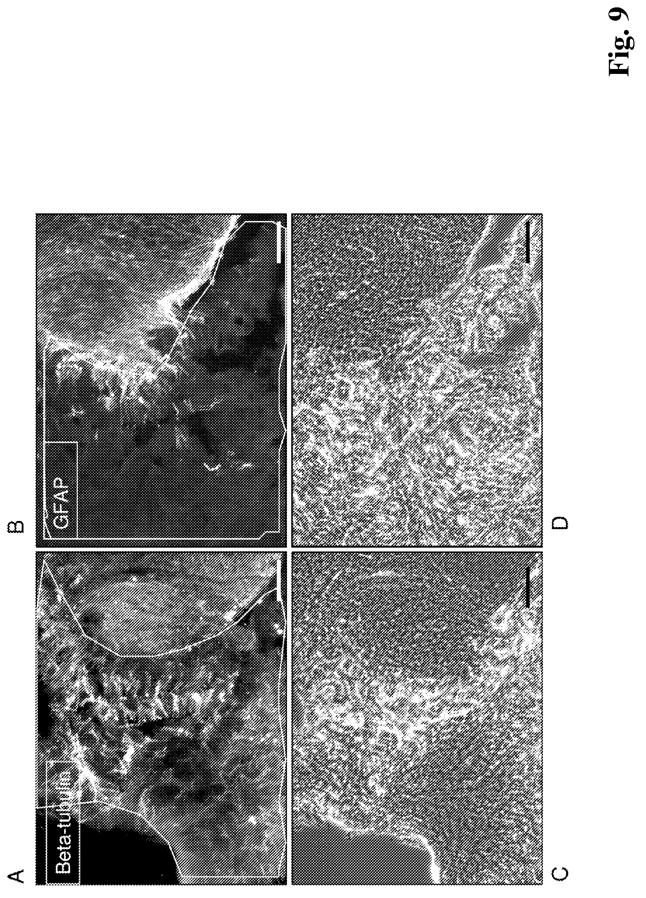

- Fig. 9: zeigt Aufnahmen einer Mikroskop-Analyse von Rückenmarksproben adulter Ratten, denen nach induzierter Rückenmarksschädigung ein (4% LVM, wässrig / 2 mmol/l CaCl₂)-Hydrogel implantiert wurde. Die behandelte Stelle (das Implantat ist durch die weiße Linie begrenzt) wurde zur Detektion von Neuriten gegen beta-Tubulin (A) und zur Detektion von Astrozyten mit einem Antikörper gegen saures Gliafaserprotein (GFAP) gefärbt (B). (C) und (D) zeigen die jeweiligen Aufnahmen im Durchlichtkanal. Die Skala entspricht 100 µm.
- Fig. 10: zeigt eine graphische Darstellung der Ergebnisse von Amplitudentests für erfindungsgemäß verwendete Hydrogele (1% LVG, wässrig / 2 mmol/l CaCl₂) und als Vergleichsbeispiel (1% LVG, wässrig / 1000 mmol/l CaCl₂). Dargestellt sind Speichermodul G' und Verlustmodul G" in Abhängigkeit der eingetragenen Schubspannung τ.
- Fig. 11: zeigt eine graphische Darstellung von Viskositätsmessungen der für die Herstellung erfindungsgemäß verwendeter Alginathydrogele eingesetzter Alginatsole vor und nach dem Autoklavieren. Die Viskosität η der Polysaccharidsole ist nach dem Autoklavieren deutlich niedriger.

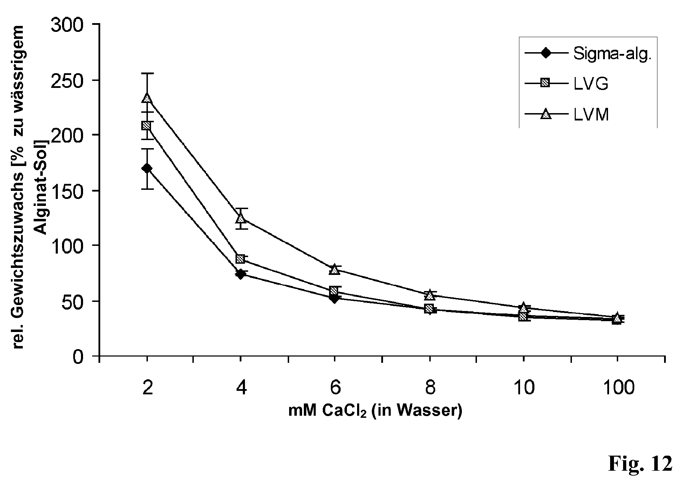

- Fig. 12: zeigte eine graphische Darstellung der Quelleigenschaften von getrockneten Polysaccharidfilmen, die nach Zugabe von wässrigen Lösungen von unterschiedlichem Gehalt an Calciumionen ein Hydrogel ausbilden. Dargestellt ist die relative Änderung des Gewichts durch die Rehydratisierung und die daraus resultierende Vernetzung (Verhältnis ,Gewicht von rehydratisiertem Hydrogel zu wässrigem Alginatsol vor dem Trocknen'). Verwendet wurde Natriumalginat der Firma Sigma-Aldrich ("Sigma-alg."), und die Alginate LVG und LVM von NovaMatrix.

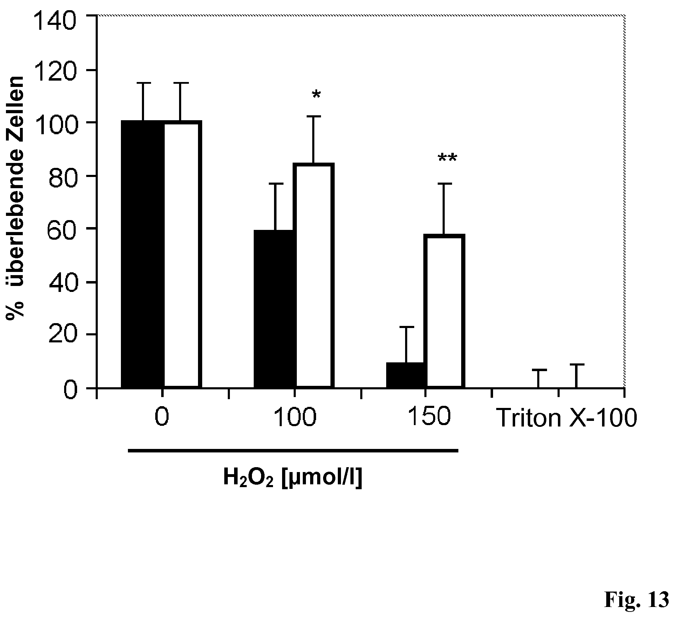

- Fig. 13: zeigt das Überleben von Neuronen in Gegenwart von Wasserstoffperoxid, also unter oxidativem Stress. Verglichen wurde das Überleben in Gegenwart von (0,2% LVM, rehydratisiert, 1,8 mmol/l CaCl₂, weiße Balken) und ohne erfindungsgemäßes Hydrogel (schwarze Balken) bei unterschiedlichen Intensitäten von oxidativem Stress.

In den Ausführungsbeispielen wurden kommerziell erhältliche Alginate der Firmen NovaMatrix und Sigma-Aldrich verwendet. Die folgende Tabelle 1 zeigt die Zusammensetzung der in den Beispielen verwendeten Alginate:

| | % ManUA |
|---|---|
| **NovaMatrix:** | |
| LVM | 57 |
| SLM20 | 57 |
| SLM100 | 57 |
| LVG | 32 |
| **Sigma Aldrich** (#71238) | unbekannt |

### Ausführungsbeispiel 1:

Das Ausführungsbeispiel 1 demonstriert, dass Hydrogele aus Alginaten, welche in Gegenwart von erfindungsgemäßen Konzentrationen multivalenter Kationen hergestellt wurden, das Neuritenwachstum fördern.

Dazu wurden Nervenzellen wie folgt isoliert: Nervenzellen wurden aus Kortizes von 18 Tage alten Wistar-Rattenföten isoliert. Das Gewebe wurde in eisgekühlter 33 mmol/l Glucose-HBSS (PAA Laboratories) aufgenommen, zerkleinert und für 30 min bei 37 °C in 0,25 %iger Trypsin-EDTA-Lösung inkubiert. Die Zellen wurden in eine gepufferte Salzlösung enthaltend 10 % fötales Rinderserum (FBS) und 0,01 % DNase l überführt und durch vorsichtiges Pipettieren vereinzelt. Danach wurden sie durch ein Zellsieb mit einer Porengröße von 70 µm gegeben und für 10 min bei 800 U/min zentrifugiert, in ein für Neuronen geeignetes Zellkulturmedium aufgenommen und in die Zellkulturplatten ausplattiert.

Die isolierten Nervenzellen wurden unter verschiedenen Zellkulturbedingungen kultiviert, dazu wurden die Zellen in einer Dichte von 1x10⁶ Zellen je 9,6 cm² ausgesät und für 72 h kultiviert. In einem Kontrollversuch wurden sie auf mit Poly-L-Lysin beschichteten Zellkulturplatten aus Kunststoff ausgesät. Um den Einfluss von mannuronsäurereichen Alginaten der Typen LVM, SLM20, SLM100 und Alginat von Sigma-Aldrich auf das Wachstum von Nervenzellen zu testen, wurden diese weiterhin auf Zellkulturplatten ausgesät, welche mit Hydrogelen aus den mannuronsäurereichen Alginaten bedeckt waren.

Diese wurden wie folgt hergestellt: Ein jeweils 2 %iges Sol von Alginaten der Typen LVM, SLM20, SLM100 und Sigma-Aldrich in Wasser wurde hergestellt. Diese wurde durch einen 0,45 µm Sterilfilter (0,45 µm Porengröße) filtriert und weiter zu einem 0,2 %igen Alginatsol verdünnt (außer Sigma-Aldrich-Alginat, **Fig. 2**)**.** Die Hydrogele wurden durch Übereinanderschichten von 5 ml Zellkulturmedium als Kationenlösung auf 500 µl des 0,2 %igen Alginatsols hergestellt.

Das verwendete Zellkulturmedium bestand aus mit B-27 ergänzten Neurobasalmedium (Life Technologies), welches 0,5 mmol/l l-Glutamin und 1 % Antibiotika-Antimykotika-Mix enthielt. Die Inkubation erfolgte bei 37 °C und 5 % CO₂. Alle vier Tage wurde das Medium zu 30% ausgetauscht.

Das eingesetzte Neurobasalmedium enthält zusätzlich zu 1,8 mmol/l Calciumionen auch Spuren weiterer Kationen (Price P.J. et al., Protocols for Neural Cell Culture, 3rd Ed., Humana Press, Inc. 2001, S. 255 ff.). Es sind im verwendeten Neurobasalmedium 1,8 mmol/l CaCl₂, 4,5·10⁻⁶ mmol/l Fe(NO₃)₃·9 H₂O und 0,00066 mmol/l ZnSO₄·7 H₂O enthalten.

Nach einer Inkubation für 10 bis 60 Minuten wurde das überschüssige Zellkulturmedium von dem Alginathydrogel, welches sich am Boden der Zellkulturplatte ausgebildet hat, entfernt. Dies lieferte (0,2 % LVM, wässrig / 1,8 mmol/l CaCl₂), (2 % Sigma-Aldrich, wässrig / 1,8 mmol/l CaCl₂) und (0,2 % SLM100, wässrig / 1,8 mmol/l CaCl₂)-Hydrogele.

Die Zellen wurden lichtmikroskopisch untersucht **(****Fig. 1****,** **Fig. 2****).** Im Vergleich zur Kultivierung auf Poly-L-Lysin konnte bei Kultivierung auf Alginat-Hydrogelen ein deutlich stärkeres Wachstum von Neuriten festgestellt werden.

### Ausführungsbeispiel 2:

Das Ausführungsbeispiel 2 zeigt das Neuritenwachstum nach Kultivierung von Nervenzellen auf Alginathydrogelen, welche aus einem vorher getrockneten Alginatfilm hergestellt wurden.

Diese wurden wie folgt hergestellt: Alginatsole von LVM und guluronsäurereichem Alginat des Typen LVG wurden, wie in Ausführungsbeispiel 1 beschrieben, hergestellt. LVM wurde in einem Versuch sterilfiltriert und in einem weiteren Versuch autoklaviert. Durch das zweite Verfahren werden die Polysaccharidketten zum Teil gekürzt.

Die 1 %igen wässrigen Sole wurden auf der Oberfläche einer 6-well Zellkulturplatte verteilt und luftgetrocknet, so dass sich auf der Oberfläche der Zellkulturplatte ein Alginatfilm ausbildete. Durch Zugabe einer Lösung mit 1,8 mmol/l CaCl₂ wurden daraus (1 % LVM, rehydratisiert / 1,8 mmol/l CaCl₂)-, (1 % LVM, autoklaviert, rehydratisiert / 1,8 mmol/l CaCl₂)-bzw. (1 % LVG, rehydratisiert / 1,8 mmol/l CaCl₂)-Hydrogele gebildet.

Die Nervenzellen wurden analog zu Ausführungsbeispiel 1 isoliert und unter ebendiesen Zellkulturbedingungen für 48 h kultiviert. Als Kontrolle wurden die Zellen ebenfalls auf Poly-L-Lysin beschichteten Zellkulturplatten kultiviert.

Die Zellen wurden anschließend lichtmikroskopisch betrachtet **(****Fig. 3** **A, B und C).** Wohingegen bei der Kultur auf Poly-L-Lysin beschichteten Zellkulturplatten eindeutig geringeres Neuritenwachstum festgestellt werden konnte **(A),** ist bei der Kultur auf einem (1 % LVM, rehydratisiert / 1,8 mmol/l CaCl₂)-Hydrogel ausgeprägtes Neuritenwachstum zu beobachten **(B, C).** Auch Hydrogele aus autoklaviertem Alginat zeigten ein gegen über der Poly-L-Lysin-Kontrolle deutlich verstärktes Neuritenwachstum.

Die statistische Auswertung der Anzahl der Neuriten auf einer definierten Fläche zeigt, dass ein Hydrogel aus mannuronsäurereichem LVM, welches unter Verwendung von Calciumionen hergestellt wurde, das Neuritenwachstum effektiver anregt, als guluronsäurereiches LVG **(****Fig. 3 D)****.** Dies kann sowohl nach 24 h als auch nach 48 h in der Zellkultur festgestellt werden.

### Ausführungsbeispiel 3:

Das Ausführungsbeispiel 3 zeigt die Kultur von isolierten, zu Sphäroiden reassemblierten Nervenzellen der Ratte bei unterschiedlichen Alginatkonzentrationen von LVG.

Nervenzellen wurden analog zu den Ausführungsbeispielen 1 und 2 isoliert und anschließend in unbeschichtete Zellkulturplatten ausgesät. Dadurch haften die Zellen nicht an der Oberfläche der Kulturplatte, sondern aneinander unter der Bildung von kugelförmigen Zellclustern. Die gebildeten Zellcluster wurden nach 72 h in Kultur isoliert und für 24 h unterschiedlich kultiviert (die Medien und Kulturbedingungen waren analog zu den Ausführungsbeispielen 1 und 2).

Als Kontrollen wurden die Zellcluster auf unbeschichteten **(****Fig. 4 A)** und mit Poly-L-Lysin beschichteten **(****Fig. 4 B)** Zellkulturplatten ausgesät. Weiterhin wurden sie auf einem (0,2 % LVG, wässrig / 1 mol/l CaCl₂)-Hydrogel kultiviert **(****Fig. 4 C)****.** In Fig. 4A und Fig. 4C konnte bei der lichtmikroskopischen Analyse kein Neuritenwachstum aus den Zellclustern festgestellt werden. Das Neuritenwachstum auf Poly-L-Lysin war gering.

Nur bei Verwendung einer niedrigen CaCl₂-Konzentration zur Bildung des Alginat-Hydrogels, wie bei dem verwendeten (0,2 % LVG, wässrig / 1,8 mmol/l CaCl₂)-Hydrogel **(****Fig. 4 D)****,** konnte dagegen ein deutlich erhöhtes Neuritenwachstum aus den Zellclustern festgestellt werden.

### Ausführungsbeispiel 4:

Das Ausführungsbeispiel 4 zeigt die Kultur von isolierten, zu Sphäroiden reassemblierten Nervenzellen der Ratte auf Alginathydrogelen, welche unter Verwendung von LVM und bei unterschiedlichen Kationenkonzentrationen hergestellt wurden.

Nervenzellen wurden analog zu den Ausführungsbeispielen 1 und 2 isoliert und anschließend in unbeschichtete Zellkulturplatten ausgesät. Dadurch haften die Zellen nicht an der Oberfläche der Kulturplatte, sondern aneinander unter der Bildung von kugelförmigen Zellclustern. Die gebildeten Zellcluster wurden nach 72 h in Kultur isoliert und für 24 h unterschiedlich kultiviert (die Medien und Kulturbedingungen waren analog zu den Ausführungsbeispielen 1 und 2).

Als Vergleichsbeispiel wurden die Zellcluster auf einem (0,2 % LVM, wässrig / 1 mol/l CaCl₂)-Hydrogel kultiviert **(****Fig. 5 A)****.** Es konnte in der lichtmikroskopischen Analyse kein Neuritenwachstum aus den Zellclustern festgestellt werden. Weiterhin wurden die Zellcluster auf einem (0,2 % LVM, wässrig / 2 mmol/l CaCl₂)-Hydrogel kultiviert **(****Fig. 5 B)****.** Dabei konnte ein deutlich erhöhtes Neuritenwachstum aus den Zellclustern festgestellt werden.

### Ausführungsbeispiel 5:

Das Ausführungsbeispiel 5 zeigt, dass die Anfälligkeit von Nervenzellen gegenüber oxidativem Stress in Gegenwart von erfindungsgemäß verwendeten Alginathydrogelen vermindert ist. Dazu wurden Nervenzellen analog zu den Ausführungsbeispielen 1 und 2 isoliert und kultiviert.

Sie wurden entweder auf mit Poly-L-Lysin beschichteten Zellkulturplatten (PLL), auf mit Poly-L-Lysin beschichteten Zellkulturplatten in der Gegenwart von 0,1 % LVM im Zellkulturmedium, wobei das LVM am 7. Kulturtag zugegeben wurde (PLL+LVM) oder auf Platten, welche mit einem (0,2 % LVM, rehydratisiert / 1,8 mmol/l CaCl₂)-Hydrogel bedeckt waren, kultiviert. Zur Induktion von oxidativem Stress wurde jeweils 100 µmol/l (schraffierte Balken) bzw. 200 µmol/l (schwarze Balken) H₂O₂ zugegeben. Im Zellkulturüberstand wurde dann die Freisetzung von Lactatdehydrogenase (LDH) als Nachweis der Zytotoxizität mit einem kommerziellen Kit (Cytotoxicity detection kit, Roche) nach 8 Tagen und 14 Tagen der Kultur bestimmt **(****Fig. 6****).** Die Analysen zeigen, dass die Anfälligkeit von Nervenzellen gegenüber oxidativem Stress in Gegenwart von den erfindungsgemäß verwendeten Hydrogelen deutlich vermindert ist.

### Ausführungsbeispiel 6:

Das Ausführungsbeispiel 6 zeigt das Wachstum von Neuriten bei Kultivierung von Nervenzellen auf (1 % LVM, wässrig / 2 mmol/l Salz)- und (1 % LVM, wässrig / 100 mmol/l Salz)-Hydrogelen (als Vergleichsbeispiele) unter Verwendung von Salzen unterschiedlicher zweiwertiger Kationen.

Dazu wurden Nervenzellen analog zu Ausführungsbeispiel 1 und 2 isoliert und kultiviert. Es wurden die folgenden Salze verwendet: CaCl₂, BaCl₂ und SrCl₂. Das Ausführungsbeispiel 6 zeigt, dass das Neuritenwachstum unabhängig von den eingesetzten multivalenten Kationen nur bei geringen Konzentrationen der Kationen im Hydrogel gefördert wird **(****Fig. 7****).** Bei geringen Konzentrationen der Salze (2 mmol/l) ist bei der Kultivierung von Nervenzellen auf Alginathydrogelen Neuritenwachstum festzustellen **(****Fig. 7 A-C****).** Bei höheren Konzentrationen der Salze (100 mmol/l) kann bei der Kultivierung von Nervenzellen auf Alginathydrogelen kein Neuritenwachstum festgestellt werden **(****Fig. 7 D-E****).** Das Ausführungsbeispiel zeigt, dass der wachstumsfördernde Effekt der Alginathydrogele nur bei geringen Konzentrationen der quervernetzenden Salze eintritt.

### Ausführungsbeispiel 7:

Das Ausführungsbeispiel 7 zeigt das Wachstum von Neuriten bei Kultivierung von Nervenzellen auf (1 % LVG, wässrig / 2 mmol/l Salz)- und (1 % LVG, wässrig / 100 mmol/l Salz)-Hydrogelen unter Verwendung von ZnCl₂ und ZnSO₄.

Dazu wurden Nervenzellen analog zu Ausführungsbeispiel 1 und 2 isoliert und kultiviert. Das Ausführungsbeispiel 7 zeigt, dass das Neuritenwachstum auch bei der Verwendung von Zn²⁺-Ionen deutlich gefördert wird **(****Fig. 8** **B, C)** im Vergleich zur Neuritenbildung bei Kultivierung auf Poly-L-Lysin beschichteten Zellkulturplatten **(****Fig. 8 A)****.** ZnCl₂ und ZnSO₄ hatten auf die Nervenzellen in der Konzentration von 100 mmol/l einen (aus der Literatur bekannten) toxischen Effekt (Daten nicht dargestellt).

### Ausführungsbeispiel 8:

Das Ausführungsbeispiel 8 zeigt die Effekte der Alginathydrogele auf das Wachstum von Neuriten nach Implantation in Rückenmarksschädigungen von adulten Ratten.

Dafür wurde ein (4 % LVM, wässrig / 2 mmol/l CaCl₂)-Hydrogel folgendermaßen hergestellt: ein steril-filtriertes wässriges 4 %iges LVM-Sol wurde mit 2 mmol/l CaCl₂ für 60 min quervernetzt und anschließend für 60 min in einer 150 mmol/l NaCl-Lösung equilibriert.

Bei 40 Tage alten Wistarratten wurden die Rückenmarksegmente T9-10 der linken Seite des Rückenmarks entfernt, so dass eine Lücke von 3 bis 4 mm Länge entstand. Die rechte Hälfte des Rückenmarks blieb intakt. Das Hydrogel wurde in die Lücke implantiert. Sechs Wochen nach der Operation wurden die Ratten anästhesiert und mit 4 %iger Formaldehydlösung in 0,1 mol/l Phosphatpuffer (pH 7,4) perfundiert. Das Rückenmark wurde isoliert und für 12 h in 10 % Saccharose und anschließend 30 % Saccharose in der Perfusionslösung inkubiert. Die Proben wurden in Kryomedium eingebettet und auf Trockeneis eingefroren. Es wurden 20 µm dicke Schnitte angefertigt, welche mit Antikörpern gegen das für Nervenzellen spezifische Protein beta-Tubulin und gegen das für Astrozyten spezifische Protein GFAP in einer Immunfluoreszenzfärbung gefärbt wurden. Anschließend wurden die Proben am Fluoreszenzmikroskop untersucht **(****Fig. 9****).** Es ist darauf zu erkennen, dass nach der Implantation des Hydrogels das Wachstum von Neuriten stattgefunden hat **(****Fig. 9 A)****.** Im Gegensatz dazu konnten keine Astrozyten detektiert werden **(****Fig. 9 B)****.** Diese Ergebnisse demonstrieren, dass mit Hilfe des Hydrogels das Neuritenwachstum angeregt wurde, eine Überbrückung des Defektes durch Neuriten erzielt wurde, ohne dass es dabei zu einer Narbenbildung gekommen ist (kein Astrozytenwachstum in das Hydrogel nachweisbar).

### Ausführungsbeispiel 9:

Im Ausführungsbeispiel 9 wurden Amplitudentests für Alginathydrogele durchgeführt. Es wurde ein erfindungsgemäß verwendetes Hydrogel (1 % LVG, wässrig / 2 mmol/l CaCl₂) und als Vergleichsbeispiel ein (1 % LVG, wässrig / 1000 mmol/l CaCl₂)-Hydrogel getestet. Als Messsystem wurde ein Rheometer (Anton Paar Physica MCR301) mit einer Platte-Platte Messzelle PP25 eingesetzt. Die Messungen wurden bei 37 °C durchgeführt.

Die getesteten Alginat-Hydrogele wurden bei konstanter Frequenz und logarithmisch steigender Amplitude oszillatorisch belastet. Das Speichermodul G' bei kleinen Schubspannungen ist ein Maß für die Ruhestruktur der Probe und liegt bei (1 % LVG, wässrig / 1000 mmol/l CaCl₂) bei G'=20,8 kPa und somit mehr als 30mal höher als bei (1 % LVG, wässrig / 2 mmol/l CaCl₂), wo ein Speichermodul G' von 0,64 kPa gemessen wurde (siehe dazu **Fig. 10**).

Im linerarviskoelastischen Bereich, also dem Bereich in dem das Speichermodul konstant zur Schubspannung ist, weist das Vergleichshydrogel (1 % LVG, wässrig / 1000 mmol/l CaCl₂) eine höhere komplexe Viskosität auf als das erfindungsgemäß verwendete (1 % LVG, wässrig / 2 mmol/l CaCl₂).

Das Vergleichshydrogel (1 % LVG, wässrig / 1000 mmol/l CaCl₂) weist ebenfalls einen höheren Wert der Schubspannung am Fließpunkt auf (dieser liegt am Schnittpunkt von G' mit dem Verlustmodul G"). Bei dem Vergleichshydrogel (1 % LVG, wässrig / 1000 mmol/l CaCl₂) ist somit eine höhere Kraft notwendig, bis das Hydrogel zu fließen beginnt.

### Ausführungsbeispiel 10:

Im Ausführungsbeispiel 10 wurden Viskositätsmessungen mit autoklavierten und nicht autoklavierten Alginatsolen durchgeführt, um die Viskositätsunterschiede zu verdeutlichen.

Es wurden 1 %ige Alginatsole von LVG und LVM der Firma NovaMatrix analysiert, jeweils unbehandelt und zum Vergleich nach dem Autoklavieren. Die Messungen wurden bei 20 °C durchgeführt.

Die Sole wurden in Rotation vermessen. Dabei wurden die Viskositätswerte η bei kontinuierlich logarithmischen steigenden Scherraten aufgenommen. Die untersuchten Sole zeigen in den Scherratenbereich von 0,1-100 s⁻¹ ein nahezu idealviskoses Fließverhalten mit der leichten Tendenz zu scherverdünnenden Verhalten, also einer Abnahme der Viskosität mit steigender Scherrate (**Fig. 11**). Für die Angabe der Viskosität wurde im Scherratenbereich von 0,1-100 s⁻¹ idealviskoses Verhalten angenommen und konstante Viskosität interpoliert. Folgende Viskositätswerte wurden ermittelt:

| | η [mPa·s] |
|---|---|
| LVG | 145,0 |
| LVG, autoklaviert | 27,4 |
| LVM | 21,2 |
| LVM, autoklaviert | 6,1 |

Nach der Behandlung im Autoklaven nimmt die Viskosität des Alginatsols ab. Dies ist auf die Verkürzung der Polysaccharidketten zurückzuführen. Die wachstumsfördernden Eigenschaften auf das Neuritenwachstum sind auch bei Hydrogelen, welche aus autoklavierten Alginatsolen hergestellt wurden, nachweisbar (**Fig. 3C**).

### Ausführungsbeispiel 11:

Es wurden Quellungseigenschaften von Alginat-Hydrogelen bei unterschiedlichen Gehalten multivalenter Kationen bestimmt. Dazu wurden jeweils 1%ige wässrige Lösungen dreier verschiedener Alginate bereitgestellt.

600 µl der jeweiligen wässrigen Alginatlösung wurde zunächst auf einer Feinwaage gewogen und anschließend für 24 h bei Raumtemperatur getrocknet, so dass ein trockener Alginatfilm entstand. Anschließend wurden jeweils 10 ml einer wässrigen Lösung, welche CaCl₂ enthielt, zugegeben, so dass ein Hydrogel ausgebildet wurde. Das Gewicht des Hydrogels wurde ebenfalla auf einer Feinwaage bestimmt. Die Gewichtsänderung bei unterschiedlichen Kationenkonzentrationen ist in Fig. 12 als das Gewichtsverhältnis der wässrigen Alginatlösung zu Hydrogel dargestellt.

Die Hydrogele-zeigten mit absteigender Kationenkonzentration einen Gewichtszuwachs (**Fig. 12**). Dies demonstriert, dass Alginat-Hydrogele mit einem Gehalt an multivalenten Kationen von maximal 4 mmol/l charakteristische Eigenschaften aufweisen, die bei Hydrogelen, welche mit höheren Kationenkonzentrationen hergestellt wurden, nicht festgestellt werden konnten.

Daraus ist abzuleiten, dass erfindungsgemäß verwendete Akginathydrogele mit Kationenkonzentrationen von weniger als 4 mmol/l mehr eingelagerte Flüssigkeit in den quervernetzen Polysaccharidketten enthalten. Ein möglicher positiver Effekt auf die aktive Förderung des Neuritenwachstums könnte daher im vergrößerten Porensystem im Polysaccharidnetzwerk liegen, was es den Neuriten ermöglicht, in das Netzwerk einzuwachsen. Durch das erweiterte Porensystem ist ebenfalls ein verbesserter Nährstoffaustausch möglich, so dass eine günstige Nährstoffversorgung der Neuriten sichergestellt ist.

### Ausführungsbeispiel 12:

Ratten-Neuronen wurden analog zu Ausführungsbeispiel 1 und 2 isoliert und kultiviert. Analog zu Ausführungsbeispiel 5 wurden die Neuronen zur Bestimmung der Anfälligkeit gegenüber oxidativem Stress in Gegenwart von wässriger Wasserstoffperoxidlösung kultiviert. Bestimmt wurde das überleben der Neuronen in Gegenwart eines erfindungsgemäßen Alginat-Hydrogels (0,2 % LVM, rehydratisiert / 1,8 mmol/l CaCl₂, **Fig. 13****,** weiße Balken) und ohne erfindungsgemäßes Hydrogel (schwarze Balken) bei unterschiedlichen Konzentrationen von H₂O₂. Während der Kultivierung wurde der Zellproliferationsindikator AlamarBlue (AbD Serotec) zugesetzt, um lebendige von apoptotischen Zellen zu unterscheiden. Die Anwesenheit des Alginat-Hydrogels vermindert den Anteil der durch oxidativen Stress abgetöteten Zellen signifikant. Um auszuschließen, dass dieser Effekt auf eine selektive Hemmung der Lactatdehydrogenaseaktivität (LDH-Aktivität) durch das Alginat-Hydrogel zurückzuführen ist, wurde in einem Vergleichsexperiment Triton X-100 zur Kultur gegeben. Triton X-100 bewirkt eine maximale LDH-Freisetzung. Sowohl mit als auch ohne Alginat-Hydrogel führte dies zur Abtötung der gesamten Zellen in der Probe. Diese Ergebnisse zeigen, dass die Anfälligkeit von Neuronen gegenüber oxidativem Stress in Gegenwart des erfindungsgemäß verwendeten Hydrogels deutlich vermindert ist und dies nicht auf eine selektive LDH-Freisetzungs-Hemmung durch das Hydrogel zurückzuführen ist.

### Ausführungsbeispiel 13:

Neuronen wurden analog zu Ausführungsbeispiel 1 und 2 isoliert und zur Kultivierung direkt auf erfindungsgemäß verwendete Alginat-Hydrogele ausgesät. Als Vergleichsbeispiel wurden die Neuronen ohne erfindungsgemäß verwendetes Hydrogel auf Poly-L-lysin-beschichteten Kunststoff-Wellplatten kultiviert und mit jeweils einem Alginat-Hydrogel der folgenden Zusammensetzung:
- 0,2 % LVM, rehydratisiert / 1,8 mmol/l CaCl₂
- 1 % LVM, wässrig / 1,8 mmol/l CaCl₂
- 1 % LVM, autoklaviert/rehydratisiert / 1,8 mmol/l CaCl₂ (in dieser Ausführungsvariante wurde das wässrige Alginatsol vor der 24-stündigen Trocknung autoklaviert, anschließend wurde wie in Punkt b) verfahren).

Das verwendete Zellkulturmedium bestand aus mit B-27 ergänzten Neurobasalmedium (Life Technologies), welches 0,5 mmol/l I-Glutamin und 1 % Antibiotika-Antimykotika-Mix enthielt. Die Inkubation erfolgte bei 37 °C und 5 % CO₂. Alle vier Tage wurde das Medium zu 30% ausgetauscht. Die Zellen wurden jeweils nach 4- und 6-tägiger Kultur mikroskopisch betrachtet und die Länge der ausgebildeten Neuriten bestimmt. Die Neuritenlänge wurde unter Liveaufnahmen im Phasenkontrast mit Hilfe der NeuronJ-Software ausgewertet. Die Neuritenlänge wurde durch Mittelwertbestimmung von mindestens zehn unabhängigen Aufnahmen einer Fläche von 1000 µm² ermittelt.

Der Vergleich der Neuritenlängen nach vier und sechstägiger Kultur zeigte, dass die Neuriten in Gegenwart erfindungsgemäß verwendeter Hydrogele mit deutlich erhöhter Geschwindigkeit gebildet werden. Es kann also ein direkter wachstumsfördernder Effekt der erfindungsgemäß verwendeten Hydrogele auf die Geschwindigkeit des Neuritenwachstums festgestellt werden.

**Tabelle 1:**

| Zellkulturoberfläche | Veränderung der Neuritenlänge am Tag 6 im Verhältnis zur Neuritenlänge am Tag 4 |
|---|---|
| PLL | 155 % |
| 0,2 % LVM, rehydratisiert / 1,8 mmol/l CaCl₂ | 235 % |
| 1 % LVM, wässrig / 1,8 mmol/l CaCl₂ | 283 % |
| 1 % LVM, thermo/rehydratisiert / 1,8 mmol/l CaCl₂ | 212 % |

## Patentansprüche

1. Verwendung eines Hydrogels, welches *α*-L-Guluronsäure (GuIUA) und *β*-D-Mannuronsäure (ManUA) haltige Polysaccharide oder deren Salze enthält, wobei die Kohlenhydrateinheiten der Polysaccharide nicht durch chemische Linker kovalent quervernetzt sind, zur in vitro Kultivierung von Zellen und/oder Geweben des Nervensystems, , **dadurch gekennzeichnet, dass** das Hydrogel einen Gehalt von zwei- oder mehrwertigen Kationen von 1 mmol/l bis 4 mmol/l aufweist.

2. Verwendung nach Anspruch 1, **dadurch gekennzeichnet, dass** das Polysaccharid ausgewählt ist aus Alginsäure und deren Salzen (Alginate).

3. Verwendung nach Anspruch 1 oder 2, **dadurch gekennzeichnet, dass** die Polysaccharide mehr Kohlenhydrateinheiten von ManUA als von GuIUA enthalten, wobei der Anteil an ManUA im Polysaccharid mindestens 20 % beträgt.

4. Verwendung nach einem der Ansprüche 1 bis 3, **dadurch gekennzeichnet, dass** die zwei- oder mehrwertigen Kationen ausgewählt sind aus Ca²⁺, Sr²⁺, Ba²⁺, Cu²⁺, Ni²⁺, Zn²⁺, Fe²⁺ und Fe³⁺.

5. Verwendung nach einem der Ansprüche 1 bis 4, **dadurch gekennzeichnet, dass** das Hydrogel unterschiedliche Kationen enthält, wobei die Gesamtkonzentration der Kationen maximal 3 mmol/l beträgt.

6. Verwendung nach einem der Ansprüche 1 bis 5, **dadurch gekennzeichnet, dass** das Hydrogel in getrockneter Form vorliegt.

7. Hydrogel, wie in einem der Ansprüche 1 bis 6 definiert, zur Verwendung in der Behandlung oder Prophylaxe von Beschädigungen des Nervensystems oder zur Verwendung in der Behandlung oder Prophylaxe von neurodegenerativen Erkrankungen.

8. Zusammensetzung enthaltend ein Hydrogel, definiert wie in einem der Ansprüche 1 bis 6 in Kombination mit pharmazeutisch akzeptablen Hilfssubstanzen zur Verwendung als pharmazeutische Zusammensetzung oder als Implantatmaterial.

9. Kit, enthaltend
a) ein Hydrogel wie in einem der Ansprüche 1 bis 6 definiert, und
b) eine wässrige Flüssigkeit enthaltend zwei- oder mehrwertigen Kationen mit einem Gehalt von maximal 4 mmol/l
zur Verwendung als pharmazeutischer Kit.

10. Zusammensetzung zur Verwendung nach Anspruch 8 oder Kit zur Verwendung nach Anspruch 9, zur Behandlung oder Prophylaxe von Beschädigungen des Nervensystems oder Behandlung oder Prophylaxe von neurodegenerativen Erkrankungen.

11. Hydrogel zur Verwendung nach Anspruch 7, Zusammensetzung oder Kit zur Verwendung nach Anspruch 10 , **dadurch gekennzeichnet, dass** die neurodegenerativen Erkrankungen oder Beschädigungen des Nervensystems ausgewählt sind aus Nervenschädigungen bei chirurgischen Eingriffen oder Unfällen/Verletzungen, Schlaganfall, ischaemischem Schlaganfall, Schädel-Hirn-Trauma, Multipler Sklerose, akuter disseminierter Enzephalomyelitis, amyotropher Lateralsklerose (ALS), Retinopathia pigmentosa, leichter kognitiver Beeinträchtigung, Alzheimer, Pick-Krankheit, Altersdemenz, progressiver supranukleären Blickparese, subkortikalen Demenzen, Morbus Wilson, Multi-Infarkt-Demenz, arteriosklerotischer Demenz, AIDS-assozüerter Demenz, zerebraler Degeneration, spinozerebellaerer Degeneration, Friedreich-Ataxie, Louis-Bar-Syndrom, Epilepsie-assozüerten Hirnschäden, Rückenmarksschäden, Restless-Legs-Syndrom, Chorea Huntington, Morbus Parkinson, Multisystematrophie, zerebraler Vaskulitis, mitochondrialen Enzephalomyopathien, neuronaler Ceroid-Lipofuszinose, spinaler Muskelatrophie, lysosomalen Speicherkrankheiten mit ZNS-Beteiligung, Leukodystrophien, Harnstoff-zyklusdefekten, hepatischer Enzephalopathie, renaler Enzephalopathie, metabolischen Enzephalopathien, Porphyrie, bakterieller oder viraler Meningitis oder Meningoenzephalitis, Prionenerkrankungen, Vergiftungen mit neurotoxischen Verbindungen, Guillain-Barre-Syndrom, chronisch inflammatorischen Neuropathien, Polymyositis, Dermatomyositis und Strahlungs-induzierten Hirn- oder Nervenschädigungen.

12. Verwendung eines Zellkulturbehälters oder Trägermaterials für die Kultivierung von Zellen, beschichtet mit einem Hydrogel wie in einem der Ansprüche 1 bis 6 definiert zur in vitro Kultivierung von Zellen des Nervensystems (einschließlich Zelllinien) und/oder Geweben des Nervensystems.

## Claims

1. Use of a hydrogel which contains polysaccharides or their salts containing α-L-guluronic acid (GuIUA) and β-D-mannuronic acid (ManUA), wherein the carbohydrate units of the polysaccharides are not covalently crosslinked via chemical linkers, for the in vitro culture of cells and/or tissues of the nervous system, **characterized in that** the hydrogel comprises a divalent or multivalent cation content of 1 mmol/L to 4 mmol/L.

2. Use as claimed in claim 1, **characterized in that** the polysaccharide is selected from alginic acid and its salts (alginates).

3. Use as claimed in claim 1 or claim 2, **characterized in that** the polysaccharides contain more carbohydrate units of ManUA than of GuIUA, wherein the proportion of ManUA in the polysaccharide is at least 20%.

4. Use as claimed in one of claims 1 to 3, **characterized in that** the bivalent or multivalent cations are selected from Ca²⁺, Sr²⁺, Ba²⁺, Cu²⁺, Ni²⁺, Zn²⁺, Fe²⁺ and Fe³⁺.

5. Use as claimed in one of claims 1 to 4, **characterized in that** the hydrogel contains different cations, wherein the maximum total concentration of the cations is 3 mmol/L.

6. Use as claimed in one of claims 1 to 5, **characterized in that** the hydrogel is in the dry form.

7. A hydrogel as defined in one of claims 1 to 6, for use in the treatment or prophylaxis of injuries to the nervous system or for use in the treatment or prophylaxis of neurodegenerative diseases.

8. A composition containing a hydrogel as defined in one of claims 1 to 6, in combination with pharmaceutically acceptable excipients for use as a pharmaceutical composition or as an implant material.

9. A kit containing
a) a hydrogel as defined in one of claims 1 to 6, and
b) an aqueous liquid containing a maximum bivalent or multivalent cation content of 4 mmol/L,
for use as a pharmaceutical kit.

10. A composition for use as claimed in claim 8 or a kit for use as claimed in claim 9, for the treatment or prophylaxis of injuries to the nervous system or for the treatment or prophylaxis of neurodegenerative diseases.

11. A hydrogel for use as claimed in claim 7, a composition or kit for use as claimed in claim 10, **characterized in that** the neurodegenerative diseases or injuries to the nervous system are selected from nerve damage during surgical interventions or accident/injuries, stroke, ischaemic stroke, traumatic brain injury, multiple sclerosis, acute disseminated encephalomyelitis, amyotrophic lateral sclerosis (ALS), retinopathia pigmentosa, slight cognitive impairment, Alzheimer's disease, Pick's disease, senile dementia, progressive supranuclear palsy, subcortical dementia, Morbus Wilson disease, multi-infarct dementia, arteriosclerotic dementia, AIDS-associated dementia, cerebral degeneration, spinocerebellar degeneration, Friedrich's ataxia, Louis-Bar syndrome, epilepsy-associated brain damage, spinal cord injuries, restless legs syndrome, Chorea Huntington's disease, Morbus Parkinson's disease, multisystem atrophy, cerebral vasculitis, mitochondrial encephalomyopathies, neuronal ceroid lipofuscinosis, spinal muscular atrophy, lysosomal storage diseases with CNS involvement, leukodystrophies, urea cycle disorders, hepatic encephalopathy, renal encephalopathy, metabolic encephalopathies, porphyria, bacterial or viral meningitis or meningoencephalitis, prion diseases, poisoning with neurotoxic compounds, Guillain-Barré syndrome, chronic inflammatory neuropathies, polymyositis, dermatomyositis and radiation-induced brain or nerve damage.

12. Use of a cell culture container or support material for the culture of cells coated with a hydrogel as defined in one of claims 1 to 6, for the in vitro culture of cells of the nervous system (including cell lines) and/or tissues of the nervous system.

## Revendications

1. Utilisation d'un hydrogel, lequel contient des polysaccharides contenant de l'acide *α*-L-guluronique (GuIUA) et *β*-D- mannuronique ou leurs sels, les unités d'hydrates de carbone des polysaccharides n'étant pas réticulées à la transversale de manière covalente par des lieurs chimiques, pour la culture in-vitro de cellules et/ou de tissus du système nerveux,
**caractérisée en ce que** l'hydrogel présente une teneur de 1 mmole/l à 4 mmoles/l de cations bivalents ou multivalents.

2. Utilisation selon la revendication 1, **caractérisée en ce que** le polysaccharide est choisi parmi l'acide alginique et ses sels (alginates).

3. Utilisation selon la revendication 1 ou la revendication 2, **caractérisée en ce que** les polysaccharides contiennent plus d'unités d'hydrates de carbone de ManUA que de GulUA, la part en ManUA dans le polysaccharide s'élevant au moins à 20 %.

4. Utilisation selon l'une quelconque des revendications 1 à 3, **caractérisée en ce que** les cations bivalents ou multivalents sont choisis parmi le Ca²⁺, le Sr²⁺, le Ba²⁺, le Cu²⁺, le Ni²⁺, le Zn²⁺, le Fe²⁺ et le Fe³⁺.

5. Utilisation selon l'une quelconque des revendications 1 à 4, **caractérisée en ce que** l'hydrogel contient différents cations, la concentration totale des cations s'élevant au maximum à 3 mmoles.

6. Utilisation selon l'une quelconque des revendications 1 à 5, **caractérisé en ce que** l'hydrogel se présente sous forme séchée.

7. Hydrogel, tel que défini dans les revendications 1 à 6, destiné à être utilisé dans le traitement ou la prophylaxie de détériorations du système nerveux ou pour être utilisé dans le traitement ou la prophylaxie de pathologies neuro-dégénératives.

8. Composition contenant un hydrogel, tel que défini dans l'une quelconque des revendications 1 à 6, en association avec des substances auxiliaires acceptables du point de vue pharmaceutique destinée à être utilisée en tant que composition pharmaceutique ou que matière d'implants.

9. Kit, contenant
a) un hydrogel, tel que défini dans l'une quelconque des revendications 1 à 6 et
b) un liquide aqueux contenant des cations bivalents ou multivalents, avec une teneur d'un maximum de 4 mmoles/l
destiné à être utilisé en tant que kit pharmaceutique.

10. Composition, destinée à l'utilisation selon la revendication 8 ou kit, destiné à l'utilisation selon la revendication 9 pour le traitement ou la prophylaxie de détériorations du système nerveux ou pour le traitement ou la prophylaxie de pathologies neuro-dégénératives.

11. Hydrogel, destiné à l'utilisation selon la revendication 7, composition ou kit destiné(e) à l'utilisation selon la revendication 10, caractérisé(e) en ce que les pathologies neuro-dégénératives ou les détériorations du système nerveux sont choisies parmi les détériorations nerveuses lors d'interventions chirurgicales ou d'accidents/de blessures, d'une attaque d'apoplexie, d'une attaque ischémique, d'un traumatisme cranio-cérébral, de scléroses multiples, d'encéphalomyélite aiguë disséminée, de sclérose latérale amyotrophique (ALS), de rétinopathie pigmentaire, de léger trouble cognitif, de maladie d'Alzheimer, de maladie de Pick, de démence sénile, de paralysie visuelle supra-nucléaire progressive, de démence sous-corticale, de Morbus Wilson, de démence par infarctus multiples, de démence artério-sclérotique, de démence associée au SIDA, de dégénération cérébrale, de dégénération spino-cérébelleuse, d'ataxie de Friedreich, de syndrome de Louis Bar, de lésions cérébrales associées à l'épilepsie, de lésion de la moelle épinière, de syndrome de jambes sans repos, de chorea Huntington, de la maladie de Parkinson, d'atrophie multisystématisée, de vasculite cérébrale, d'encéphalo-myopathies mitochondriales, de céroïde-lipofuscinose neuronale, d'atrophie musculaire spinale, de maladies de surcharges lysosomales affectant le SNC, de leucodystrophies, de défauts de fonctionnement du cycle de l'urée, d'encéphalopathie hépatique, d'encéphalopathie rénale, d'encéphalopathie métabolique, de porphyrie, de méningite bactérienne ou virale ou de méningo-encéphalite, des maladies à prions, d'empoisonnements par composés neurotoxiques, de syndrome de Guillain Barré, de neuropathies inflammatoires chroniques, de polymyosite, de dermatomyosite et de détériorations cérébrales et nerveuses induites par des rayonnements.

12. Utilisation d'un récipient pour la culture de cellules ou d'une matière porteuse pour la culture de cellules, revêtu(e) d'un hydrogel, tel que défini dans l'une quelconque des revendications 1 à 6, pour la culture in-vitro de cellules du système nerveux (y compris de lignées cellulaires) et/ou de tissus du système nerveux.
